# EUROPÄISCHE PATENTSCHRIFT

(11) **EP 0 548 710 B1**
(45) Veröffentlichungstag und Bekanntmachung des Hinweises auf die Patenterteilung: **16.10.1996**
(21) Anmeldenummer: 92121142.1
(22) Anmeldetag: 11.12.1992
(51) Int. Cl.: C07D 491/048, A01N 43/90

(54) **Glykolaldehyd- und Milchsäurederivate, deren Herstellung und herbizide Verwendung**
Glycol aldehyde and lactic acid derivatives, their preparation and their use as herbicides
Dérivés d'aldéhyde glycolique et d'acide lactique, leur préparation et utilisation comme herbicides

(30) Priorität: 21.12.1991 DE 4142570
(43) Veröffentlichungstag der Anmeldung: 30.06.1993
(73) Patentinhaber: BASF Aktiengesellschaft, 67063 Ludwigshafen (DE)
(72) Erfinder: Rheinheimer, Joachim, Dr., W-6700 Ludwigshafen (DE); Baumann, Ernst, Dr., W-6720 Speyer (DE); Vogelbacher, Uwe Josef, Dr., W-6700 Ludwigshafen (DE); Saupe, Thomas, Dr., W-6902 Sandhausen (DE); Bratz, Matthias, Dr., W-6720 Speyer (DE); Meyer, Norbert, Dr., W-6802 Ladenburg (DE); Gerber, Matthias, Dr., W-6703 Limburgerhof (DE); Westphalen, Karl-Otto, Dr., W-6720 Speyer (DE); Walter, Helmut, Dr., W-6719 Obrigheim (DE); Kardorff, Uwe, Dr., W-6800 Mannheim 1 (DE)

(56) Entgegenhaltungen:
- EP-A- 0 400 741
- EP-A- 0 490 224

## Beschreibung

Die vorliegende Erfindung betrifft Glykolaldehyd- und Milchsäurederivate sowie deren Schwefelanaloge der Formel I 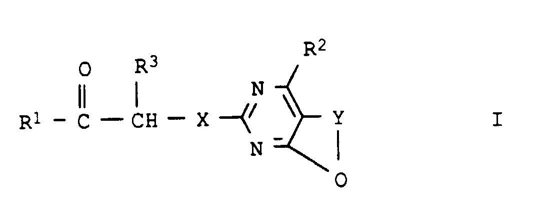 in der die Substituenten folgende Bedeutung haben:
- R¹: Wasserstoff;
eine Succinylimidoxygruppe;
ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
ein Rest 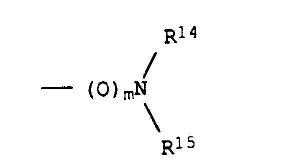 in dem m für 0 oder 1 steht und R¹⁴ und R¹⁵, die gleich oder unterschiedlich sind, die folgende Bedeutung haben: Wasserstoff;
C₁-C₆-Alkyl, C₃-C₆-Alkinyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkinyloxy, C₃-C₆-Alkinyloxy, C₁-C₆-Alkylthio, C₃-C₆-Alkinylthio, C₃-C₆-Alkinylthio, C₁-C₆-Halogenalkoxy, Cyano, C₁-C₆-Alkylcarbonyl, C₃-C₆-Alkinylcarbonyl, C₃-C₆-Alkinylcarbonyl, C₁-C₆-Alkoxycarbonyl, C₃-C₆-Alkinyloxycarbonyl, C₃-C₆-Alkinyloxycarbonyl, bis-C₁-C₆-Dialkylamino, cyclo-C₁-C₆-Alkyl, optionell substituiertes Phenyl;
optionell substituiertes cyclo-C₃-C₆-Alkyl;
optionell substituiertes Phenyl;
oder R¹⁴ mit R¹⁵ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;

R¹ ferner eine Gruppe 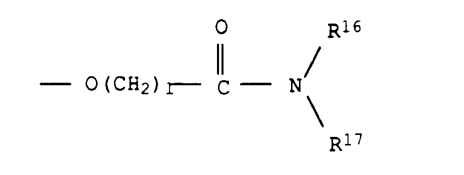
R¹ ferner in der R¹⁶ und R¹⁷, die gleich oder unterschiedlich sind, für Wasserstoff, C₁-C₆-Alkyl, optionell substituiertes Phenyl, C₃-C₆-Alkinyl oder C₃-C₆-Alkinyl stehen können und l die Werte 1, 2, 3 oder 4 annimmt;
eine Gruppe 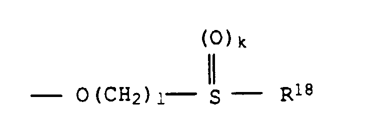 in der R¹⁸ für C₁-C₆-Alkyl, optionell substituiertes Phenyl, C₁-C₆-Halogenalkyl, C₃-C₆-Alkinyl oder C₃-C₆-Alkinyl steht, l die Werte 1, 2, 3 oder 4 und k die Werte 0, 1 oder 2 annehmen kann;

R¹ ferner einen Rest OR⁵, worin R⁵ bedeutet:
a) Wasserstoff, ein Alkalimetallkation, das Äquivalent eines Erdalkalimetallkations, das Ammoniumkation oder ein organisches Ammoniumion;
b) eine C₃-C₁₂-Cycloalkylgruppe, welche ein bis drei C₁-C₄-Alkylreste tragen kann;
c) eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₃-C₁₂-Cycloalkyl, C₁-C₈-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkythio;
d) eine C₁-C₁₀-Alkylgruppe, welche ein bis fünf Halogenatome tragen kann und einen der folgenden Reste trägt: ein 5-gliedriger Heteroaromat enthaltend ein bis drei Stickstoffatome, oder ein 5-gliedriger Heteroaromat enthaltend ein Stickstoffatom und ein Sauerstoff- oder Schwefelatom, welche ein bis vier Halogenatome und/oder ein bis zwei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
e) eine C₂-C₆-Alkylgruppe, welche in der 2-Position einen der folgenden Reste trägt: C₁-C₆-Alkoxyimino, C₃-C₆-Alkinyloxyimino, C₃-C₆-Halogenalkenyloxyimino oder Benzyloxyimino;
f) eine C₃-C₆-Alkinyl- oder eine C₃-C₆-Alkinylgruppe, wobei diese Gruppen ihrerseits ein bis fünf Halogenatome tragen können;
g) ein Phenylrest, welcher ein bis fünf Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
h) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
i) eine Gruppe -N=CR⁶R⁷, worin R⁶ und R⁷ bedeuten:
   C₁-C₂₀-Alkyl, welches seinerseits einen Phenylrest, eine C₁-C₄-Alkoxy- und/oder eine C₁-C₄-Alkylthiogruppe tragen kann;
   Phenyl;
   oder R⁶, R⁷ gemeinsam eine C₃-C₁₂-Alkylenkette, welche ein bis drei C₁-C₃-Alkylgruppen tragen kann

R¹ ferner ein Rest 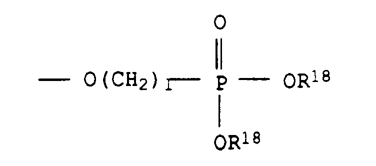 in dem R¹⁸ und l die oben genannte Bedeutung haben,

R¹ ferner ein Rest 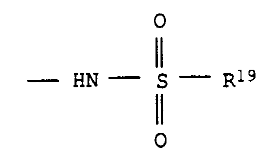 in dem R¹⁹ für die Reste C₁-C₆-Alkyl oder Phenyl steht, die ihrerseits ein bis vier der folgenden Substituenten tragen können:
Halogen, Nitro, Cyano, C₁-C₆-Alkyl;
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- R³: Wasserstoff;
eine C₁-C₈-Alkyl-, C₂-C₈-Alkinyl-, C₂-C₈-Alkinyl-, Phenyl-, C₃-C₈-Cycloalkenyl- oder C₃-C₈-Cycloalkylgruppe, die jeweils ein bis fünf Halogenatome und unabhängig voneinander ein bis drei der folgenden Substituenten tragen können:
i) Hydroxy, C₁-C₄-Halogenalkyl, C₁-C₄-Alkoxy, C₁-C₄-Alkylthio, Cyano, Nitro, C₁-C₄-Alkoxycarbonyl, C₁-C₄-Alkylcarbonyl, C₁-C₄-Alkyl, Phenylcarbonyl, C₃-C₁₂-Cycloalkyl, C₃-C₁₂-Cycloalkenyl;
ii) einen 5-gliedrigen Heterocyclus, enthaltend keine, eine oder zwei Doppelbindungen, sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkoxy, C₁-C₄-Alkylthio, C₁-C₄-Halogenalkyl oder Phenyl, das seinerseits ein bis drei Halogenatome und/oder ein bis drei Methylgruppen tragen kann;
iii) einen Thienylrest, der ein bis drei Halogenatome und/oder einen bis drei der folgenden Reste tragen kann: C₁-C₄-Alkyl, C₁-C₂-Halogenalkyl oder Nitro;
iv) einen Pyridylrest, der ein bis drei Halogenatome und/oder einen bis drei der folgenden Reste tragen kann: C₁-C₄-Alkyl, C₁-C₂-Halogenalkyl oder Nitro;
v) einen Naphthyl-, Chinolin-, Benzoxazolyl-, Benzthiazolyl-, Benzthienyl-, Indazolyl- oder Benztriazolylrest, welcher jeweils ein bis drei Halogenatome und/oder einen bis drei der folgenden Reste tragen kann: C₁-C₄-Alkyl oder C₁-C₂-Halogenalkyl;
vi) einen Phenylrest, der seinerseits ein bis fünf Halogenatome und/oder ein bis drei der folgenden Reste tragen kann: C₁-C₄-Alkyl, C₁-C₄-Alkoxy, C₁-C₄-Halogenalkyl, C₁-C₄-Halogenalkoxy, Cyano, Nitro, C₁-C₄-Dialkylamino, und/oder C₁-C₄-Alkylthio oder Nitro;
vii) einen 5- oder 6-gliedrigen Heterocyclus, enthaltend keine, eine oder zwei Doppelbindungen sowie ein bis zwei Sauerstoff- oder Schwefelatome, der außerdem folgende Reste tragen kann: C₁-C₄-Alkyl, C₁-C₄-Alkoxy, C₁-C₄-Halogenalkyl, C₁-C₄-Halogenalkoxy oder Nitro;
- R³: ferner einen 5- oder 6-gliedrigen Heterocyclus, enthaltend keine, eine oder zwei Doppelbindungen, sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl, C₁-C₄-Halogenalkoxy, C₁-C₄-Alkoxy oder Phenyl, das seinerseits ein bis drei Halogenatome und/oder ein bis drei Methylgruppen tragen kann;
einen Pyridylrest, der ein bis drei Halogenatome und/ oder einen bis drei der folgenden Reste tragen kann: C₁-C₄-Alkyl, C₁-C₂-Halogenalkyl oder Nitro;
einen Naphthyl-, Chinolin-, Benzoxazolyl-, Indazolyl- oder Benztriazolylrest, welcher jeweils ein bis drei Halogenatome und/oder einen bis drei der folgenden Reste tragen kann: C₁-C₄-Alkyl oder C₁-C₂-Halogenalkyl;
einen 5- oder 6-gliedrigen Heterocyclus, enthaltend keine, eine oder zwei Doppelbindungen sowie ein bis zwei Sauerstoff- oder Schwefelatome, der außerdem folgende Reste tragen kann: Halogen, Nitro, C₁-C₄-Alkyl, C₁-C₄-Alkoxy, C₁-C₄-Halogenalkyl, C₁-C₄-Halogenalkoxy;

R³ gemeinsam mit R¹ eine optionell substituierte C₃-C₅-Alkylenkette, in der eine CH₂-Gruppe durch Sauerstoff, Schwefel oder Stickstoff ersetzt sein kann;
- X: ein Sauerstoffatom, ein Schwefelatom oder eine Einfachbindung;
- Y: eine C₂-C₄-Alkylen oder C₂-C₄-Alkenylenkette, wobei eine Methylengruppe jeweils durch eine Oxo-Gruppe (=O) substituiert sein kann und/oder die Alkylen- bzw. Alkenylenkette durch C₁-C₄-Alkyl, Phenyl, C₁-C₄-Alkoxy oder C₁-C₄-Alkoxycarobnyl substituiert sein kann;

wobei in den oben genannten Fällen der Ausdruck optionell substituiert jeweils bedeutet, daß die so bezeichneten Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio,
sowie umweltverträgliche Salze der Verbindungen I.

Weiterhin betrifft die Erfindung Verfahren zur Herstellung der Verbindung I, sowie ihre Verwendung als Herbizide und Wachstumsregulatoren.

In der Literatur (EP-A 347 811, EP-A 400 741, EP-A 422 751 und EP-A 409 368) sind herbizid wirksame Glykolaldehyd- und Milchsäurederivate sowie deren Schwefelanaloge beschrieben. Ihre Wirkung ist jedoch oftmals unbefriedigend.

Daher war es ein Ziel, neue Glykolaldehyd- und Milchsäurederivate sowie deren Schwefelanaloge mit verbesserten herbiziden Eigenschaften sowie mit pflanzenwachstumsregulierenden Eigenschaften zu finden.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Glykolaldehyd- und Milchsäurederivate der vorstehend definierten allgemeinen Formel I ausgezeichnete pflanzenwachstumsregulierende Eigenschaften besitzen. Gegenstand der Erfindung sind neben den racemischen Verbindungen I auch die optisch aktiven (R- bzw. S-Konfiguration) wenn R³ + Wasserstoff ist.

Verbindungen der Formel I erhält man beispielsweise, indem man ein entsprechend substituiertes Glykolaldehyd- und Milchsäurederivat der Formel II mit einer entsprechenden Verbindung der Formel III in Gegenwart einer Base umsetzt. 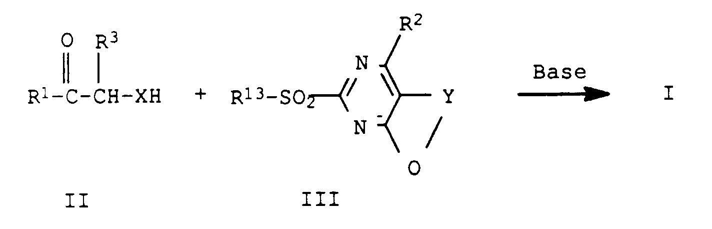 R¹³SO₂ in Formel III bedeutet eine übliche nucleofuge Abgangsgruppe, beispielsweise Arylsulfonyl wie Phenyl- oder substituiertes Phenylsulfonyl, wobei als Substituenten ein oder mehrere, z.B. 1 bis 3 niedermolekulare Alkyl- oder Alkoxyreste wie C₁-C₄-Alkyl oder Alkoxy oder Halogen, z.B. Chlor, Fluor oder Brom in Betracht kommen; oder Alkylsulfonyl wie C₁-C₄-Alkylsulfonyl, z.B. Methylsulfonyl oder Halogenalkylsulfonyl. Als Base können Alkali- oder Erdalkalimetallhydride wie NaH und CaH2, Alkalimetallhydroxide wie NaOH und KOH, Alkalimetallalkohole wie Kaliumtert.-butylat, Alkalimetallcarbonate wie Na₂CO₃ und K₂CO₃, Alkalimetallamide wie NaNH₂ und Lithiumdiisopropylamid oder tertiäre Amine Verwendung finden. Bei Einsatz einer anorganischen Base kann man einen Phasentransferkatalysator zusetzen, wenn dies den Umsatz fördert.

Die Zwischenprodukte der Formel II sind in vielen Fällen bekannt oder können nach üblichen Methoden, ausgehend von bekannten Vorprodukten hergestellt werden (vgl. z.B. EP-A 347 811, EP-A 400 741, EP-A 422 751 und EP-A 409 368).

Die Sulfone der allgemeinen Formel III erhält man, indem man ein entsprechendes 2-Alkylthio-5,6-dihydrofuran[2,3]pyrimidin (s. Collect. Czech. Chem. Commun. 32, 1582 (1967)) 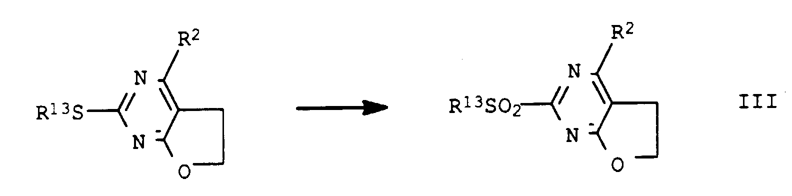 durch Oxidationsmittel wie z.B. Chlor in Wasser oder Wasserstoffperoxid in Eisessig oxydiert unter milden Bedingungen.

Die Herstellung anellierter Pyrimidine ist weiterhin beispielsweise beschrieben in 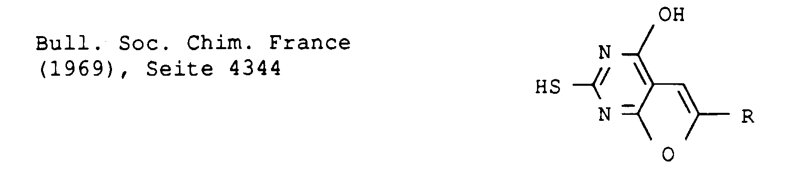 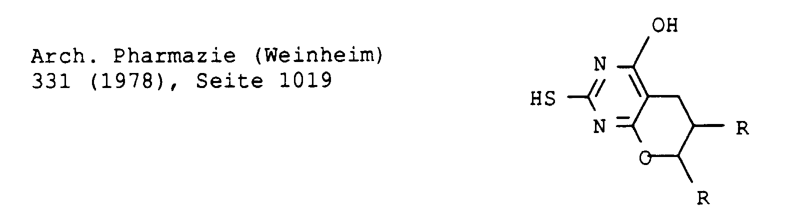 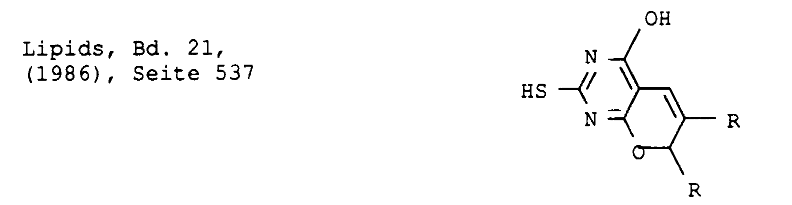 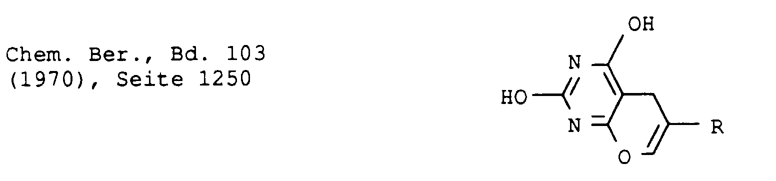 Verbindungen der Formel I können auch dadurch hergestellt werden, daß man von den entsprechenden Carbonsäuren, d.h. Verbindungen der Formel I, in denen R¹ Hydroxyl bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR¹ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d.h. von Verbindungen der Formel I, in denen R¹ für OM steht, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und erfordert wieder oftmals die Zugabe einer Base, wobei die oben genannten in Betracht kommen.

Im Hinblick auf die herbizide Wirksamkeit sind Verbindungen I bevorzugt, bei denen die Substituenten folgende Bedeutung haben:

- R¹: Wasserstoff;
eine Succinylimidoxygruppe;
ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
Alkoxy wie vorstehend genannt, mit ein bis vier Kohlenstoffatomen, Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
einen Rest 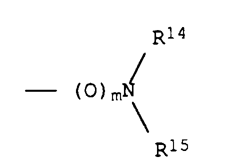 in dem m für 0 oder 1 und R¹⁴ und R¹⁵, die gleich oder unterschiedlich sein können, die folgende Bedeutung haben:
Wasserstoff;
Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl;
Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl;
wobei diese Alkyl-, Alkinyl- oder Alkinylgruppen jeweils ein bis fünf Halogenatome, besonders Chlor oder Fluor und/oder ein bis zwei der folgenden Gruppen tragen können:
C₁-C₆-Alkoxy, wie vorstehend genannt, C₃-C₆-Alkenyloxy, C₃-C₆-Alkinyloxy, C₁-C₆-Alkylthio, C₃-C₆-Alkenylthio, C₃-C₆-Alkinylthio, wobei die in diesen Resten vorliegenden den Alkyl-, Alkenyl- und Alkinylbestandteile vorzugsweise den bei R¹ im einzelnen genannten Bedeutungen entsprechen;
C₁-C₆-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
Cyano;
C₁-C₆-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,1,2-Trimethylpropylcarbonyl, 1-Ethyl-1-methylpropylcarbonyl und 1-Ethyl-2-methylpropylcarbonyl;
C₁-C₆-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl;
C₃-C₆-Alkenyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise wie voranstehend im einzelnen aufgeführt, definiert sind;
bis-C₁-C₆-Dialkylamino wie insbesondere Dimethylamino, Diethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
cyclo-C₃-C₆-Alkyl wie Cyclopropyl, Cyclobutyl, Cyclopentyl, Cyclohexyl;
optionell substituiertes Phenyl wie insbesondere Phenyl, 2-Chlorphenyl, 3-Chlorphenyl, 4-Chlorphenyl, 2-Fluorphenyl, 3-Fluorphenyl, 4-Fluorphenyl, 2-Methylphenyl, 3-Methylphenyl, 4-Methylphenyl, 2-Trifluormethylphenyl, 3-Trifluormethylphenyl, 4-Trifluormethylphenyl, 4-Methoxyphenyl, 3-Methoxyphenyl, 2-Methoxyphenyl;
optionell substituiertes cyclo-C₃-C₆-Alkyl; wie vorstehend im einzelnen genannt, beispielsweise 1-Methylthiocyclopropyl, 1-Methylcyclohexyl, 1-Methylcyclopropyl, 1-Methoxycyclohexyl,
R¹⁴ mit R¹⁵ gemeinsam eine zu einem Ring geschlossene optionell substituierte C₄-C₇-Alkylenkette oder gemeinsam eine zu einem Ring geschlossene optionell substituierte C₃-C₆-Alkylenkette mit einem Heteroatom, ausgewählt aus der Gruppe Sauerstoff, Schwefel oder Stickstoff wie -(CH₂)₄-, -(CH₂)₅-, -(CH₂)₆-, -(CH₂)₇-, -(CH₂)₂-O-(CH₂)₂-, -CH₂-O(CH₂)₃-, -(CH₂)₂-S-(CH₂)₂-, -CH₂-S-(CH₂)₃-, -CH₂-O-(CH₂)₂-, -CH₂-S-(CH₂)₂-, -(CH₂)₂-NH-(CH₂)₂-, -(CH₂)₂-N(CH₃)-(CH₂)₂-;
   R¹ ferner eine Gruppe 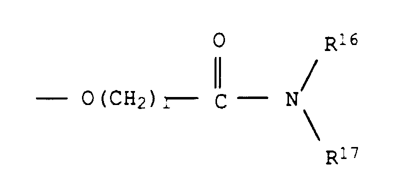 in der R¹⁶ und R¹⁷, die gleich oder unterschiedlich sind, für Wasserstoff, C₁-C₆-Alkyl, C₃-C₆-Alkenyl oder C₃-C₆-Alkinyl, jeweils wie vorstehend für R¹⁴/R¹⁵ genannt oder optionell substituiertes Phenyl stehen und l die Werte 1, 2, 3 oder 4 annimmt;
R¹ ferner eine Gruppe 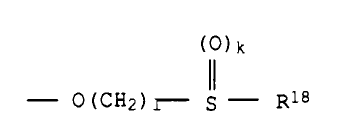 in der R¹⁸ für C₁-C₆-Alkyl, optionell substituiertes Phenyl, C₁-C₆-Halogenalkyl, C₃-C₆-Alkinyl oder C₃-C₆-Alkinyl, jeweils wie voranstehend für R¹⁴/R¹⁵ im einzelnen genannt, steht, l die Werte 1, 2, 3 oder 4 und k die Werte 0, 1 oder 2 annehmen;
   R¹ ferner einen Rest OR⁵, worin R⁵ bedeuten kann:
   Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion oder Ammonium [NH₄^{⊕}];
   ein C₃-C₁₂-Cycloalkyl, insbesondere C₃-C₆-Cycloalkyl wie Cyclopropyl, Cyclobutyl, Cyclopentyl, Cyclohexyl, das gegebenenfalls durch ein bis drei C₁-C₄-Alkylreste substituiert ist;
   C₁-C₁₀-Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl; n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, welches ein bis fünf der vorstehend genannten Halogenatome, insbesondere Fluor und Chlor und/oder einen der folgenden Reste tragen kann:
   C₁-C₄-Alkoxy, C₁-C₄-Alkylthio, Cyano, C₁-C₈-Alkylcarbonyl, C₃-C₁₂-Cycloalkyl, C₁-C₈-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkythio;
   eine C₁-C₁₀-Alkylgruppe wie vorstehend für R⁵ genannt, welche ein bis fünf Halogenatome, insbesondere Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff oder Schwefelatom, welche ein bis vier Halogenatome und/oder ein bis zwei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl,
   5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   eine C₂-C₆-Alkylgruppe, welche in der 2-Position einen der folgenden Reste trägt: C₁-C₆-Alkoxyimino, C₃-C₆-Alkinyloxyimino, C₃-C₆-Halogenalkenyloxyimino oder Benzyloxyimino;
   eine C₃-C₆-Alkinyl- oder eine C₃-C₆-Alkinylgruppe, wobei diese Gruppen ihrerseits ein bis fünf Halogenatome tragen können;
   ein Phenylrest, welcher ein bis fünf Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   eine Gruppe -N=CR⁶R⁷, worin R⁶ und R⁷ bedeuten:
   unverzweigtes oder verzweigtes C₁-C₂₀-Alkyl, vorzugsweise C₁-C₁₅-Alkyl, insbesondere C₁-C₉-Alkyl, welches einen Phenyl, einen C₁-C₄-Alkoxy und/oder einen C₁-C₄-Alkylthiotest tragen kann; Phenyl oder gemeinsam C₃-C₁₂-Alkylen, vorzugsweise C₄-C₇-Alkylen, welches ein bis drei C₁-C₃-Alkylgruppen, vorzugsweise Methyl- oder Ethylgruppen tragen kann.
   R¹ ferner ein Rest 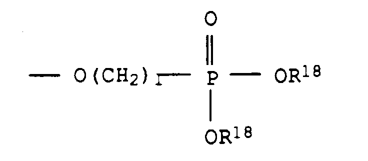 in dem R¹⁸ und l die oben genannte Bedeutung haben,
   oder R¹ ein Rest 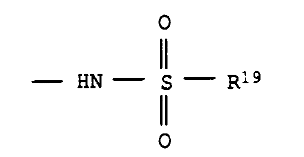 in dem R¹⁹ für die Reste C₁-C₆-Alkyl oder Phenyl steht, die ihrerseits ein bis vier der folgenden Substituenten tragen können: Halogen, Nitro, Cyano, C₁-C₆-Alkyl;

- R²: Halogen, wie Fluor, Chlor, Brom, insbesondere Fluor oder Chlor, C₁-C₄-Alkyl, C₁-C₄-Halogenalkyl, C₁-C₄-Alkoxy, C₁-C₄-Halogenalkoxy oder C₁-C₄-Alkylthio wie vorstehend im Fall von R¹⁴/R¹⁵ im einzelnen genannt;
- R³: Wasserstoff;
eine C₁-C₈-Alkyl-, C₁-C₈-Alkinyl-, C₁-C₈-Alkinyl-, Phenyl-, C₃-C₈-Cycloalkenyl- oder C₃-C₈-Cycloalkylgruppe, die jeweils bis zu 5 Halogenatome und unabhängig voneinander bis zu 3 der folgenden Substituenten tragen können: Hydroxy, C₁-C₄-Halogenalkyl, C₁-C₄-Alkoxy, C₁-C₄-Alkylthio, Cyano, Nitro, C₁-C₄-Alkoxycarbonyl, C₁-C₄-Alkylcarbonyl, C₁-C₄-Alkyl, Phenylcarbonyl, C₃-C₁₂-Cycloalkyl, C₃-C₁₂-Cycloalkenyl;
einen 5-gliedrigen Heterocyclus, enthaltend keine, eine oder zwei Doppelbindungen sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkoxy, C₁-C₄-Alkylthio, C₁-C₄-Halogenalkyl oder Phenyl, das seinerseits ein bis drei Halogenatome und/oder ein bis drei Methylgruppen tragen kann, beispielsweise seien genannt:
3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl, 3-Isopropylisoxazolin-5-yl, 3-Ethylisoxazolin-5-yl, 3-Phenylisoxazolin-5-yl, 3-tert.-Butylisoxazolin-5-yl, 4-Phenylthiazol-2-yl, 4-Phenyloxazol-2-yl, 4,5-Dimethylthiazol-2-yl, 4,5-Dimethyloxazol-2-yl, 3-Methyl-4-phenylthiazol-2-yl, 4-Methyl-3-phenylthiazol-2-yl, 3-Methyl-4-phenyloxazol-2-yl, 4-Methyl-3-phenyloxazol-2-yl, 5-Phenyl[1,3,4]oxadiazol-2-yl, 1-Pyrazolyl, 3-Methyl-1-pyrazolyl,4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 1-Imidazolyl, [1,2,4]Triazol-1-yl;
einen Thienylrest, der ein bis drei Halogenatome und/oder einen bis drei der folgenden Reste tragen kann: C₁-C₄-Alkyl, C₁-C₂-Halogenalkyl oder Nitro;
einen Pyridylrest, der ein bis drei Halogenatome und/oder einen bis drei der folgenden Reste tragen kann: C₁-C₄-Alkyl, C₁-C₂-Halogenalkyl oder Nitro;
einen Naphthyl-, Chinolin-, Benzoxazolyl-, Benzthiazolyl-, Benzthienyl-, Indazolyl- oder Benztriazolylrest, welcher jeweils ein bis drei Halogenatome und/oder einen bis drei der folgenden Reste tragen kann: C₁-C₄-Alkyl oder C₁-C₂-Halogenalkyl; einen Phenylrest, der seinerseits ein bis fünf Halogenatome und/oder ein bis drei der folgenden Reste tragen kann: C₁-C₄-Alkyl, C₁-C₄-Alkoxy, C₁-C₄-Halogenalkyl, C₁-C₄-Halogenalkoxy, Cyano, Nitro, C₁-C₄-Dialkylamino, und/oder C₁-C₄-Alkylthio;
einen 5- oder 6-gliedrigen Heterocyclus enthaltend keine, eine oder zwei Doppelbindungen sowie ein bis zwei Sauerstoff- oder Schwefelatome, der außerdem folgende Reste tragen kann: C₁-C₄-Alkyl, C₁-C₄-Alkoxy, C₁-C₄-Halogenalkyl, C₁-C₄-Halogenalkoxy; beispielsweise seien genannt: Tetrahydropyran-4-yl, Tetrahydrothiopyran-3-yl, Tetrahydropyran-3-yl, [2,6]-Dithiacyclohexyl, [2,5]-Dithiacyclopentyl, [2,6]-Dioxacyclohexyl, [2,5]-Dioxacyclopentyl, 1-Methyl-[2,6]-dithiacyclohexyl, Dihydropyran-3-yl;
- R³: einen 5- oder 6-gliedrigen Heterocyclus, enthaltend keine, eine oder zwei Doppelbindungen sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl, C₁-C₄-Halogenalkoxy, C1-C4-Alkoxy oder Phenyl, das seinerseits ein bis drei Halogenatome und/oder ein bis drei Methylgruppen tragen kann; beispielsweise seien folgende Heterocyclen aufgeführt: 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-t.-Butylisoxazol-5-yl, 3-Isopropylisoxazolin-5-yl, 3-Ethylisoxazolin-5-yl, 3-Phenylisoxazolin-5-yl, 3-tert.-Butylisoxazolin-5-yl, 4-Phenylthiazol-2-yl, 4-Phenyloxazol-2-yl, 4,5-Dimethylthiazol-2-yl, 4,5-Dimethyloxazol-2-yl, 3-Methyl-4-phenylthiazol-2-yl, 4-Methyl-3-phenylthiazol-2-yl, 3-Methyl-4-phenyloxazol-2-yl, 4-Methyl-3-phenyloxazol-2-yl, 5-Phenyl[1,3,4]oxadiazol-2-yl, 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 1-Imidazolyl, [1,2,4]Triazol-1-yl, Morpholin-1-yl, 3,5-Dimethylmorpholin-1-yl, 1-Piperidyl;
einen Pyridylrest, der ein bis drei Halogenatome und/oder einen bis drei der folgenden Reste tragen kann: C₁-C₄-Alkyl, C₁-C₂-Halogenalkyl oder Nitro;
einen Naphthyl-, Chinolin-, Benzoxazolyl, Indazolyloder Benztriazolylrest, welcher jeweils ein bis drei Halogenatome und/oder einen bis drei der folgenden Reste tragen kann: C₁-C₄-Alkyl oder C₁-C₂-Halogenalkyl;
einen 5- oder 6-gliedrigen Heterocyclus enthaltend keine, eine oder zwei Doppelbindungen sowie ein bis zwei Sauerstoff- oder Schwefelatome, wie Tetrahydropyran-4-yl, Tetrahydrothiopyran-3-yl, Tetrahydropyran-3-yl, [2,6]-Dithiacyclohexyl, [2,5]-Dithiacyclopentyl, [2,6]-Dioxacyclohexyl, [2,5]-Dioxacyclopentyl, 1-Methyl-[2,6]-dithiacyclohexyl, Dihydropyran-3-yl;
wobei der Heterocyclus außerdem folgende Reste tragen kann: Halogen, Nitro, C₁-C₄-Alkyl, C₁-C₄-Alkoxy, C₁-C₄-Halogenalkyl, C₁-C₄-Halogenalkoxy;
R³ gemeinsam mit R¹ eine ggf. substituierte C₃-C₅-Alkylenkette, in der eine CH₂-Gruppe durch Sauerstoff, Schwefel oder Stickstoff ersetzt sein kann wie -(CH₂)₃-, -(CH₂)₄-, -(CH₂)₅, -(CH₂)₂-O-, -(CH₂)₃-O-, -C(CH₃)₂-O-, -CH(CH₃)-(CH₂)₂-O-, -(CH₂)₃-NH-, -(CH₂)₄-N(CH₃)-, -(CH₂)₃-S-, -(CH₂)₄-S-, -C(CH₃)₂-CH₂)-S;
- X: ein Sauerstoffatom, ein Schwefelatom oder eine Einfachbindung; im letztgenannten Fall ist der CH(R³)-Rest direkt am Pyrimidylrest gebunden;
- Y: eine C₂-C₄-Alkylen oder C₂-C₄-Alkenylenkette, in der jeweils eine Methylengruppe durch eine Oxo-Gruppe substituiert sein kann, wie -CH₂-CH₂-, -(CH₂)₃-, -(CH₂)₄-, -CH=CH-, -CH₂-CO-, -CO-CH₂-, -CH₂-CH₂-CO-, -CH=CH-CO-, oder in der die Alkylen- bzw. Alkenylenkette durch C₁-C₄-Alkyl, Phenyl, C₁-C₄-Alkoxy oder C₁-C₄-Alkoxycarbonyl substituiert sein kann.

Die Verwendung des Ausdrucks "optionell substituiert" bedeutet jeweils, daß die so bezeichneten Gruppen einen oder mehrere, z.B. 1 bis 3 der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

Als Salze der Verbindungen I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium-, Phosphonium-, Tetraalkylammoniumsalze, Benzyltrialkylammoniumsalze, Trialkylsulfoniumsalze oder Trialkylsulfoxoniumsalze in Betracht.

Besonders bevorzugt sind Verbindungen der Formel I, bei denen
- R²: Methoxy oder Ethoxy,
- X: ein Sauerstoffatom und
- Y: eine Ethylengruppe bedeuten
und die übrigen Reste die oben ausgeführte Bedeutung haben.

Ferner sind besonders bevorzugt Milchsäurederivate der Formel I, in der R¹ eine Gruppe OR⁵ bedeutet und R⁵ für Wasserstoff, C₁-C₁₀-Alkyl, Benzyl, C₃-C₆-Alkenyl oder C₃-C₆-Alkinyl steht, R² Methoxy, R³ Wasserstoff oder C₁-C₈-Alkyl, das wie in Anspruch 1 genannt substituiert sein kann, X Sauerstoff oder Schwefel und Y eine C₂H₄-Kette bedeuten.

Weiterhin sind besonders bevorzugt Milchsäurederivate der Formel I, in der R¹ eine Gruppe OR⁵ bedeutet und R⁵ für eine Gruppe -N=CR⁶R⁷ steht, in der R⁶ bzw. R⁷ einen C₁-C₄-Alkylrest, der unsubstituiert oder durch Phenyl, C₁-C₄-Alkoxy und/oder C₁-C₄-Alkylthio substituiert ist, oder einen Phenylrest darstellt oder R⁶ zusammen mit R⁷ eine C₃-C₆-Alkylenkette bildet, die durch C₁-C₃-Alkyl substituiert sein kann, R² Methoxy, R³ Wasserstoff oder C₁-C₈-Alkyl, das wie in Anspruch 1 genannt substituiert sein kann, X Sauerstoff oder Schwefel und Y eine C₂H₄-Kette bedeuten.

Beispiel für bevorzugte Verbindungen sind in der nachfolgenden Tabelle aufgeführt:

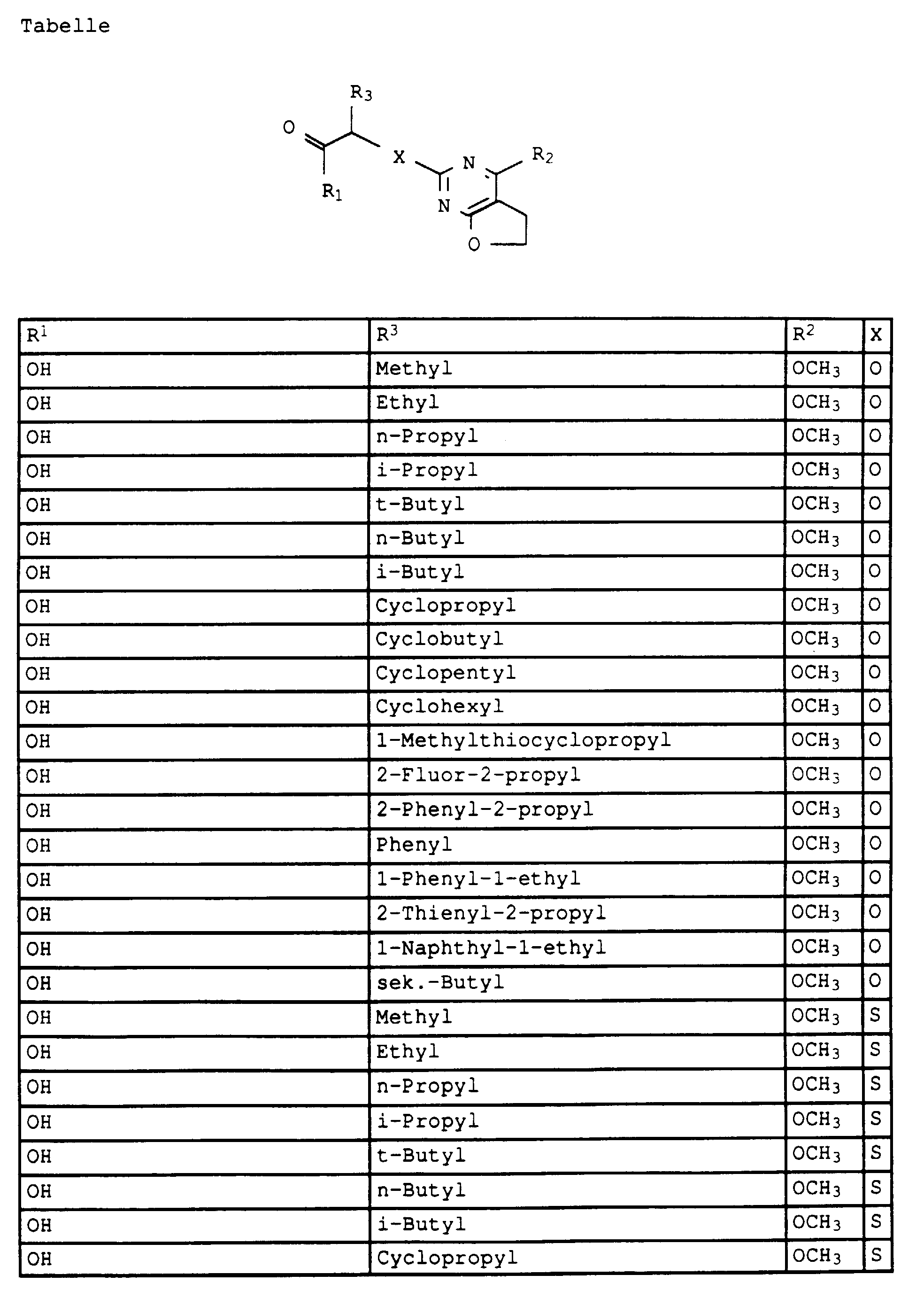

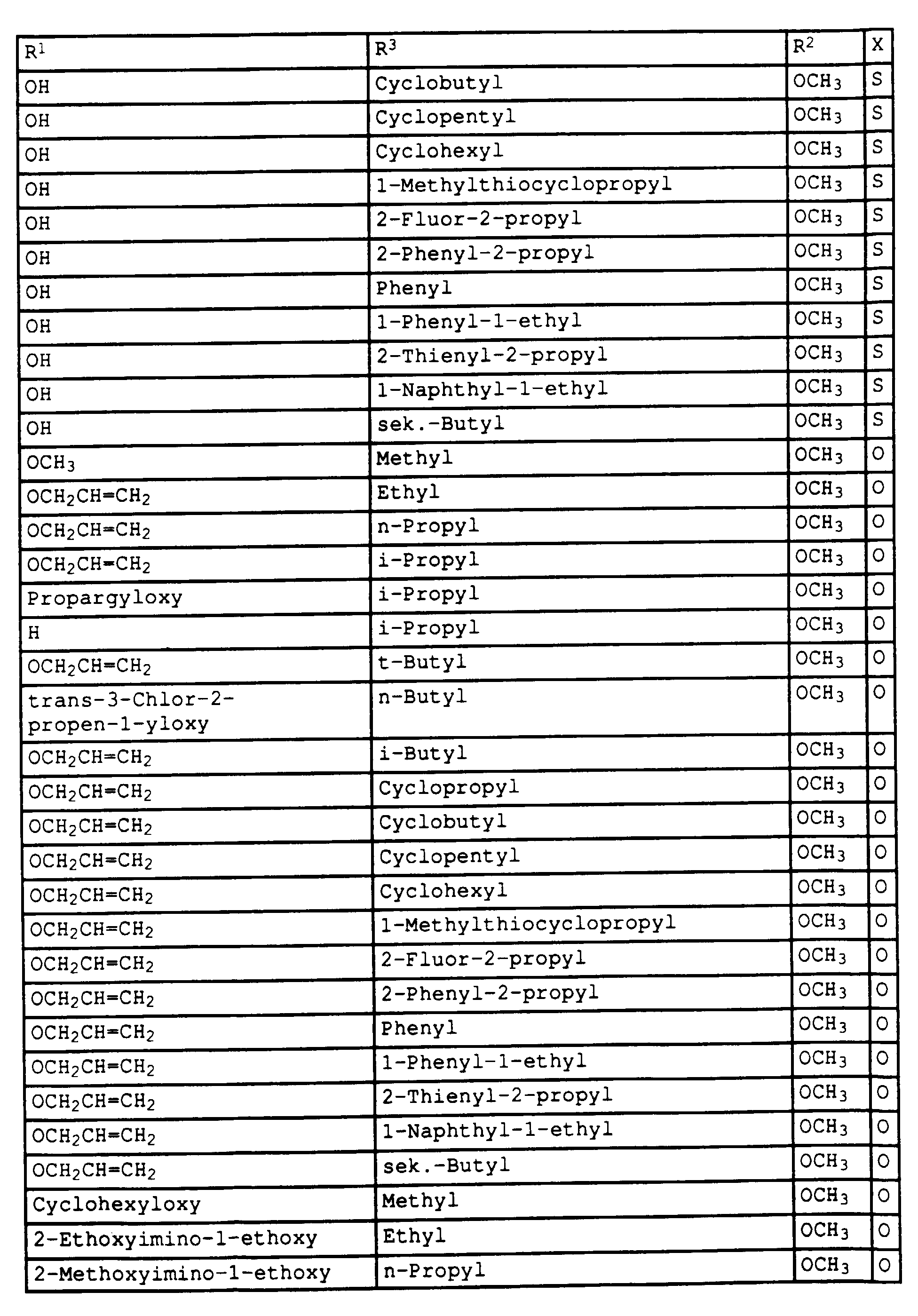

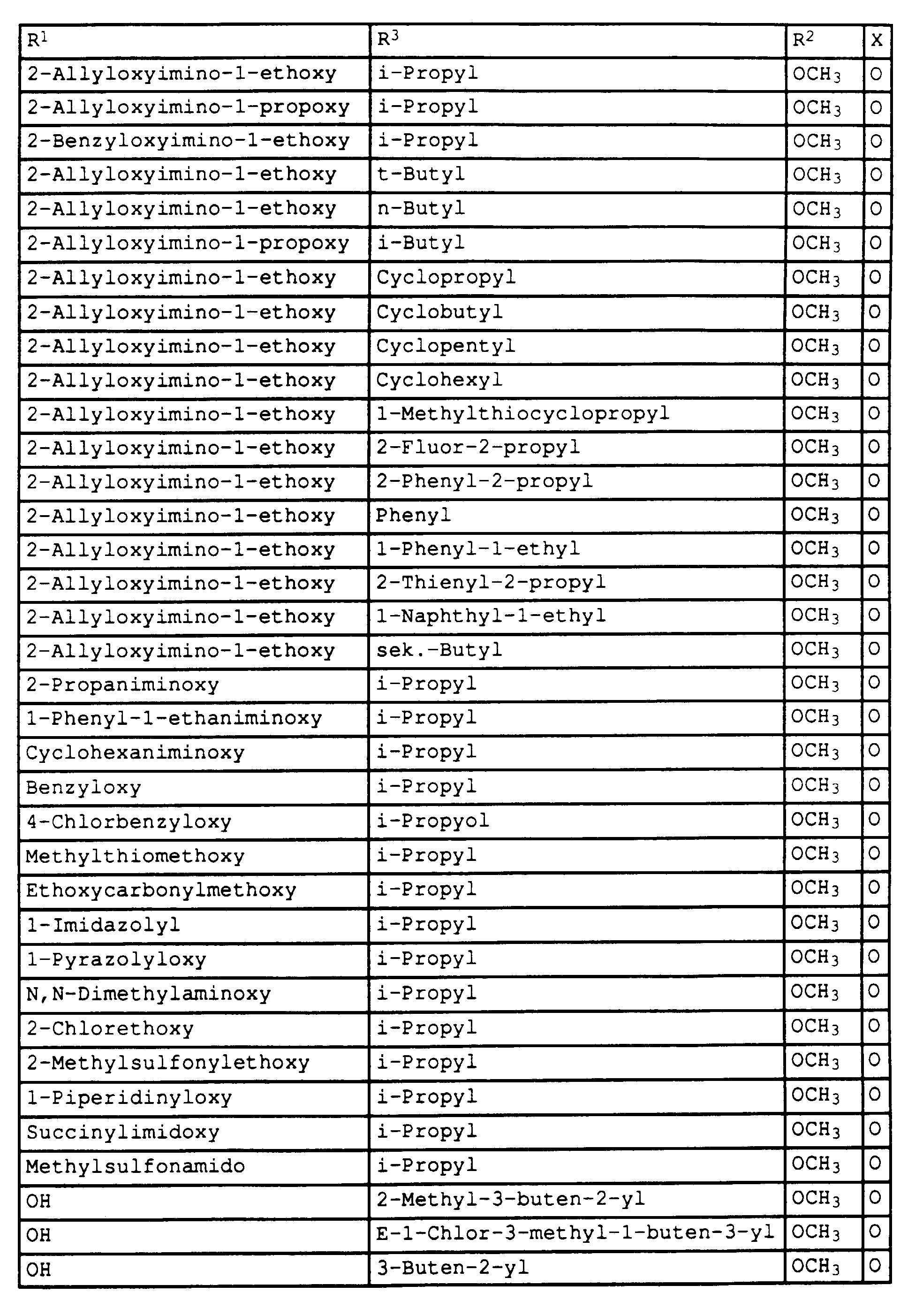

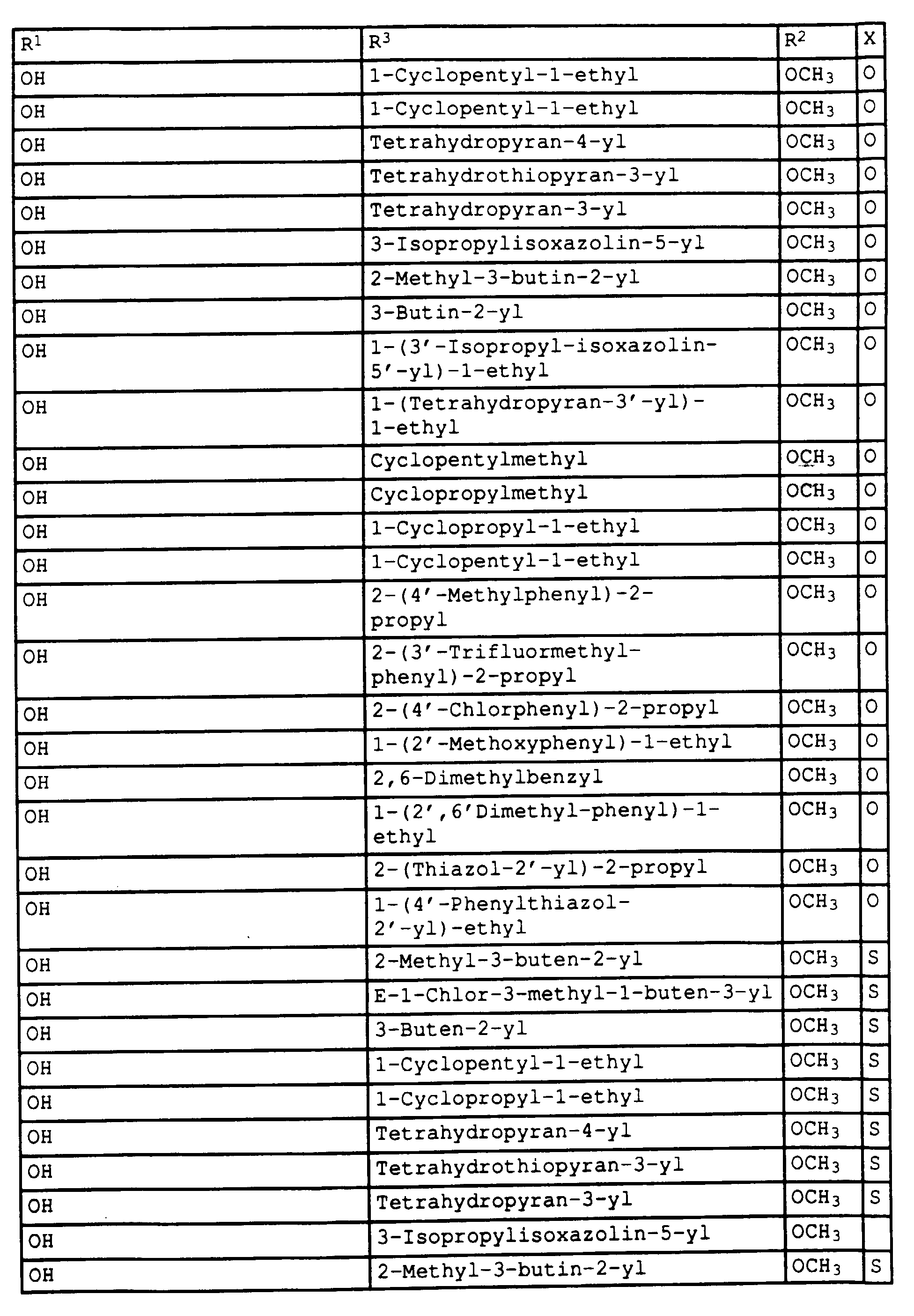

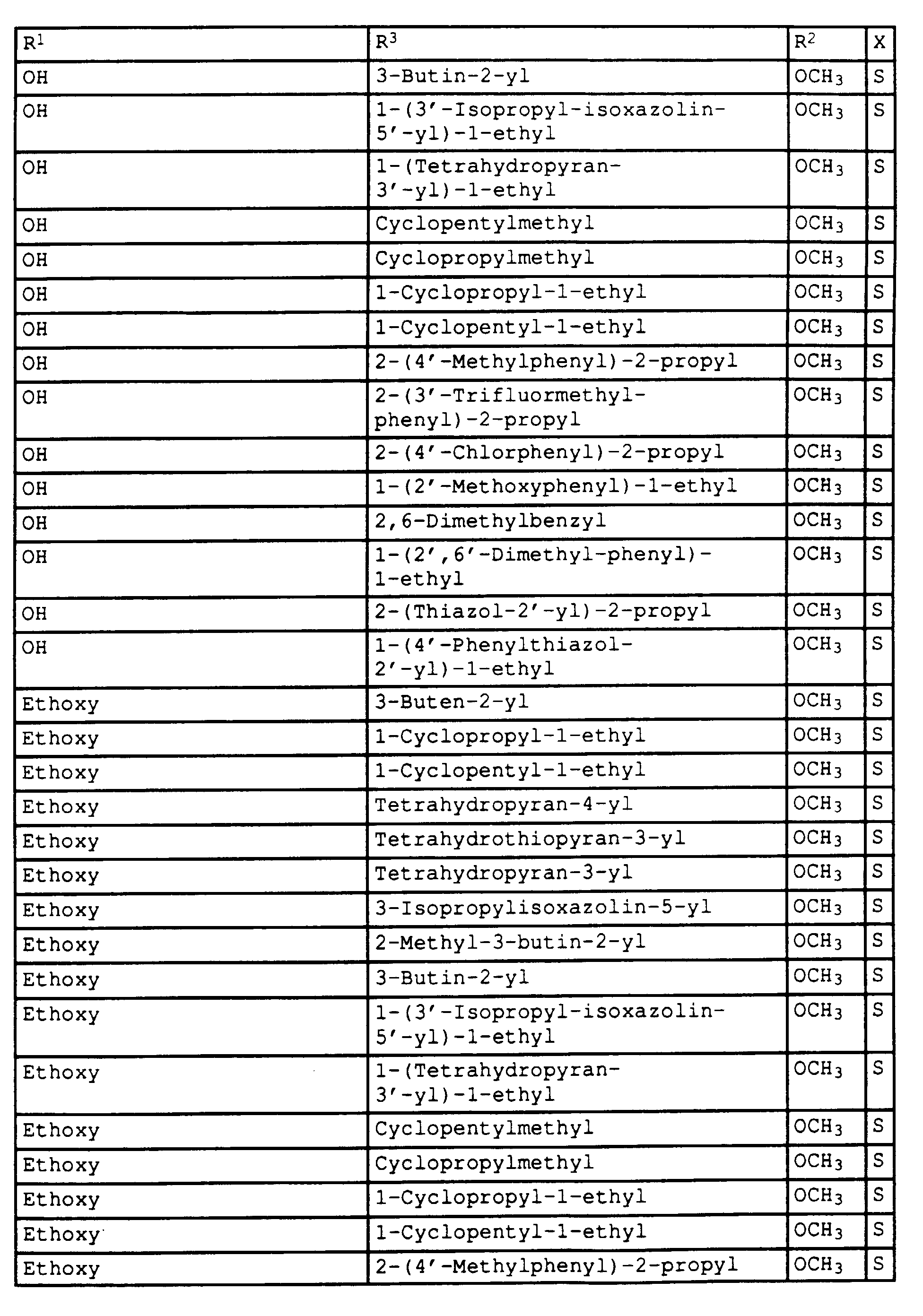

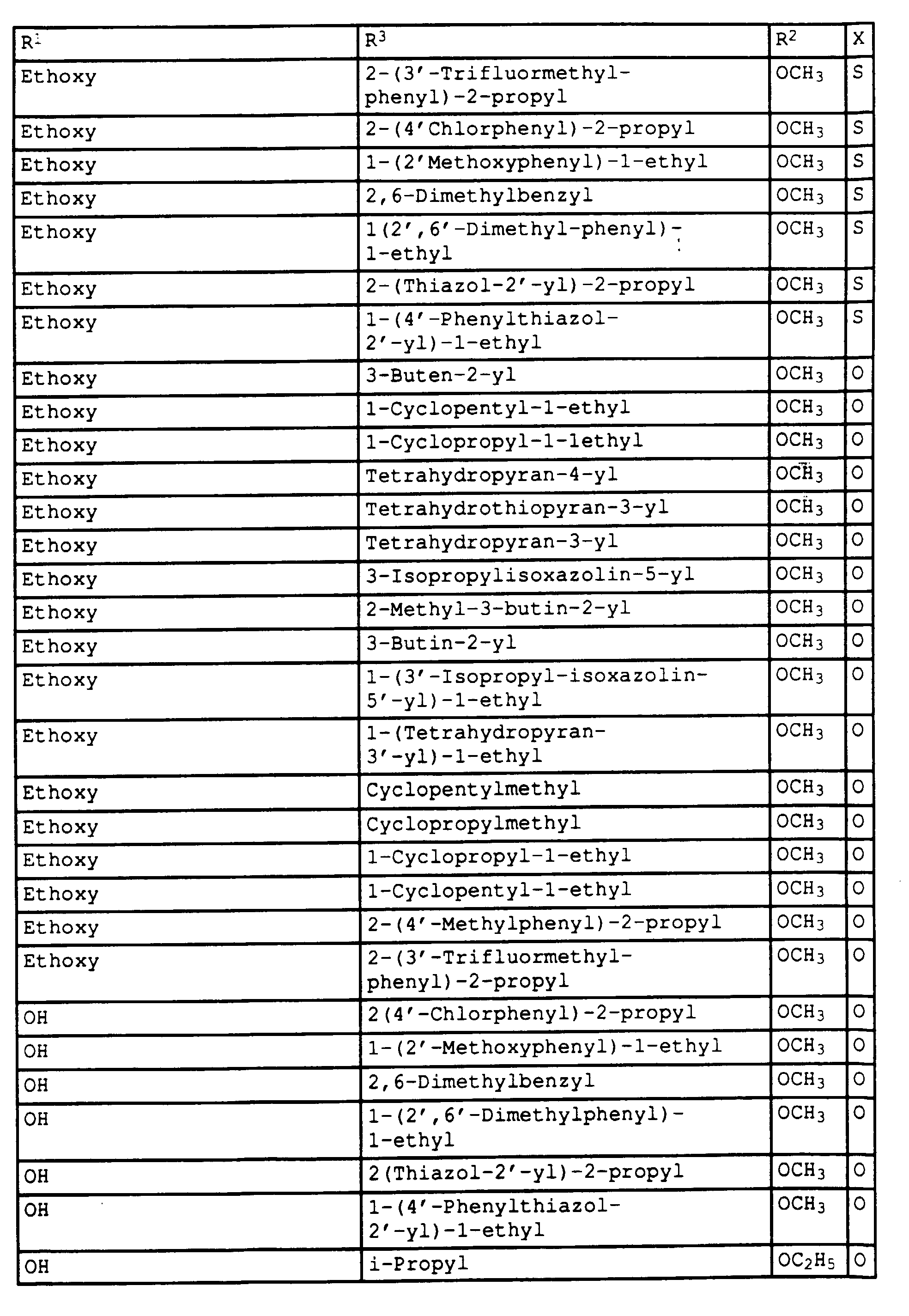

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Losungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (nach NMR-Spektrum) eingesetzt.

Die erfindungsgemäßen Verbindungen I können beispielsweise wie folgt formuliert werden:
I. Man vermischt 90 Gew.-Teilen der Verbindung Nr. 1 mit 10 Gew.-Teilen N-Methyl-α-pyrrolidon und erhält eine Lösung, die zur Anwendung in Form kleinster Tropfen geeignet ist;
II. 20 Gew.-Teile der Verbindung Nr. 5 werden in einer Mischung gelöst, die aus 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl besteht. Durch Ausgießen und feines Verteilen der Lösung in 100.000 Gew.-Teilen Wasser erhält man eine wäßrige Dispersion, die 0,02 Gew.-% des Wirkstoffs enthält.
III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
IV. 20 Gew.-Teile des Wirkstoffs Nr. 5 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
V. 20 Gew.-Teile des Wirkstoffs Nr. 1 werden mit 3 Gew.-Teilen des Natriumsalzes der Diisobutylnaphtalin-α-sulfonsäure, 1 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.
VI. 3 Gew.-Teilen des Wirkstoffs Nr. 1 werden mit 97 Gew.-Teilen feinteiligem Kaolin vermischt. Man erhält auf diese Weise ein Stäubemittel, das 3 Gew.-% des Wirkstoffs enthält.
VII. 30 Gew.-Teilen des Wirkstoffs Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gew.-Teile des Wirkstoffs Nr. 1 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenolsulfonsäureharnstoff-formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0 vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

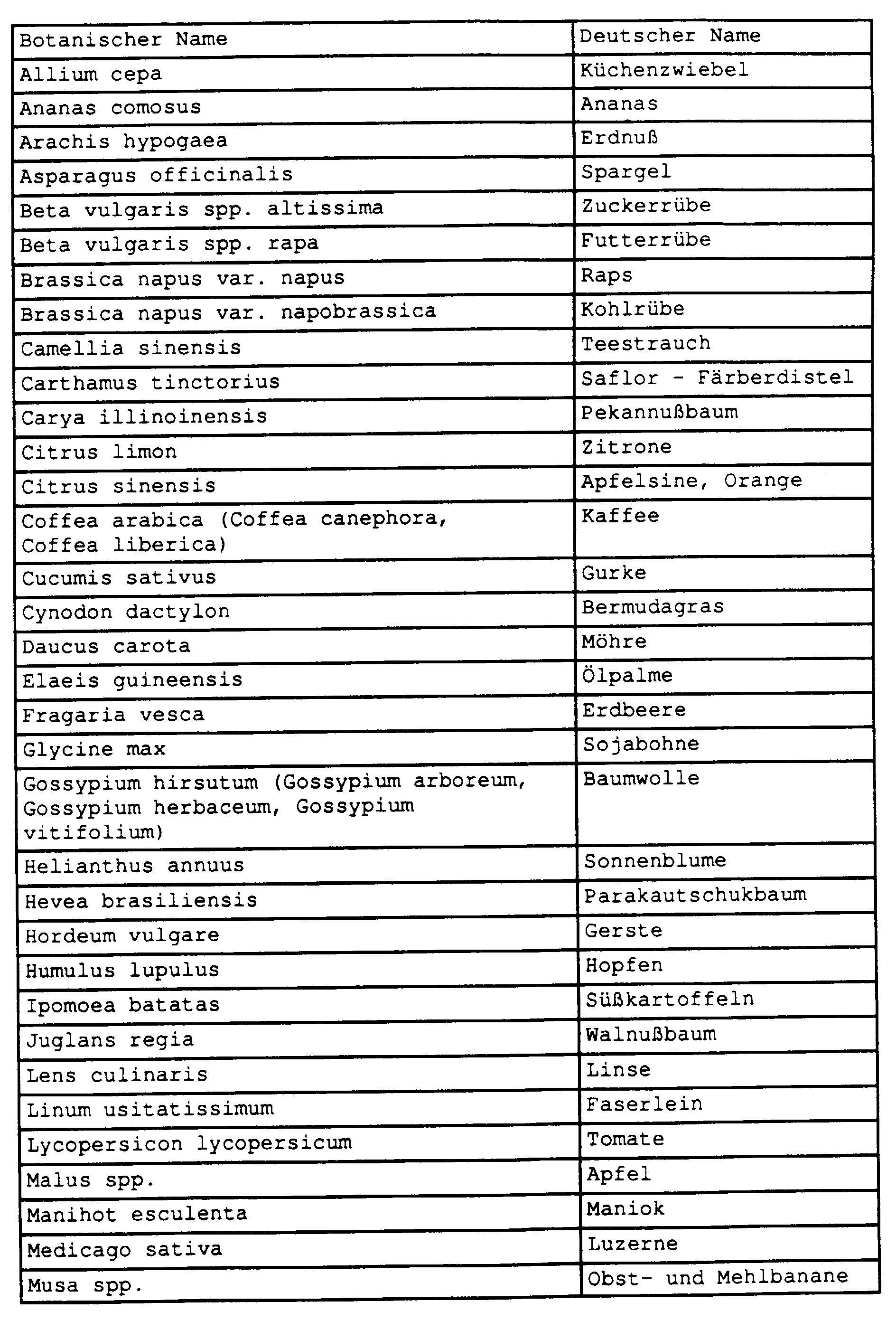

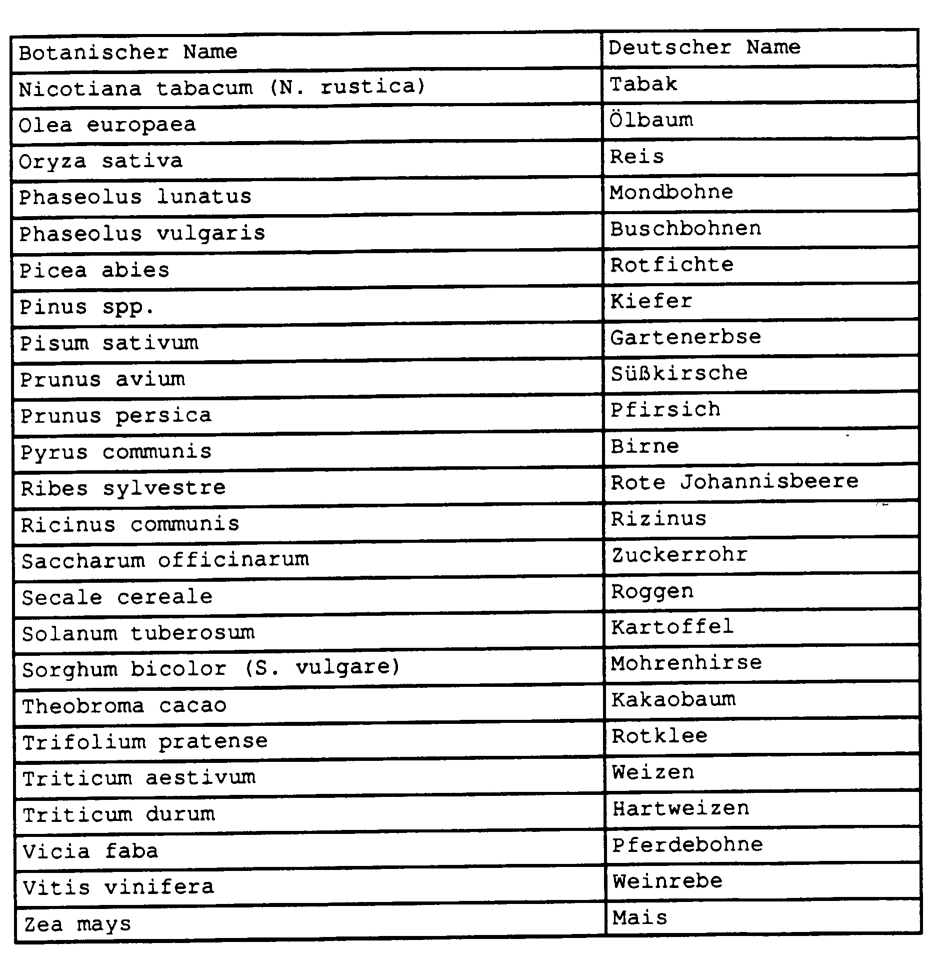

Zur Verbreiterung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Beispiel 1

### Herstellung von 2-Methylsulfonyl-4-methoxy-5,6-dihydrofuran[2,3-d]pyrimidin

### 2-Methylthio-4-chlor-5,6-dihydrofuran[2,3-d]pyrimidin

Zu einer Suspension von 65,8 g (0,357 mol) 2-Methylthio-4-hydroxy-5,6-dihydrofuran[2,3-d]pyrimidin (Collect. Czech. Chem. Commun. 32, 1582 (1967)) in 900 ml Chlorbenzol tropft man bei 125-130°C 212,0 g (1,07 mol) Trichlormethylchlorformiat in 3 Std. zu, wobei dreimal je 0,5 ml DMF zugesetzt wird. Nach 1stündigem Rühren bei 130°C wird das Reaktionsgemisch im Vakuum eingeengt und der Rückstand (74 g Öl) an Kieselgel chromatographiert (Toluol-Cyclohexan-Gemisch 9:1). Ausbeute: 17,0 g des o.g. Products vom Fp. 68-71°C.

### 2-Methylthio-4-methoxy-5,6-dihydrofuran[2,3-d]pyrimidin

17,0 g (84 mmol) 2-Methylthio-4-chlor-5,6-dihydrofuran[2,3-d]pyrimidin werden in 90 ml Methanol gegeben, bei 45°C 21,1 g (0,117 mol) 30 %ige Natriummethylat-Lösung zugetropft und 2 Std. bei 50°C nachgerührt. Nach Neutralisieren auf pH 6 mit etwas Eisessig wird das Reaktionsgemisch in 350 ml Eiswasser eingerührt. Nach Absaugen, Waschen mit Wasser und Trocknen erhält man 15,1 g des o.g. Produkts vom Fp. 90-92°C.

### 2-Methylsulfonyl-4-methoxy-5,6-dihydrofuran[2,3-d]pyrimidin

In eine Mischung von 15,1 g (76 mmol) 2-Methylthio-4-methoxy-5,6-dihydrofuran[2,3-d]pyrimidin in 120 ml Methylenchlorid und 76 ml Wasser leitet man bei 0 bis 5°C unter Rühren Chlor ein, bis die Reaktionsmischung blaß gelb gefärbt ist. Nach 30minütigem Nachrühren wird die organische Phase abgetrennt und die Wasserphase mit 100 ml Methylenchlorid extrahiert. Die vereinigten organischen Phasen werden getrocknet und eingeengt. Aus dem Rückstand (16,7 g) isoliert man nach Chromatographie an Kieselgel (Toluol-Essigester-Gemisch 4:1) 5,5 g des o.g. Produkts vom Fp. 122-24°C.

### Beispiel 2

### Herstellung von 2-Methylsulfonyl-4-methyl-5,6-dihydrofuran[2,3-d]pyrimidin

Analog zu Beispiel 1 erhält man aus 2-Methylthio-4-methyl5,6-dihydrofuran[2,3-d]pyrimidin (Collect. Czech. Chem. Commun. 32, 1582 (1967)) das obige Produkt vom Fp. 85-90°C in 80 % Ausbeute.

In entsprechender Weise sind die in Tabelle 2 aufgeführten Sulfone III erhältlich.

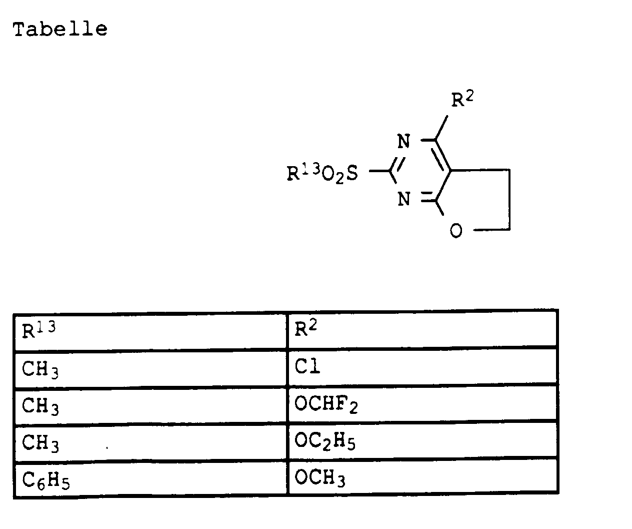

### Beispiel 3

Allgemeine Vorschrift zur Herstellung von Verbindungen der Formel I aus 2-Methylsulfonyl-4-methyl-5,6-dihydrofuran[2,3-d]pyrimidin und Milchsäurederivaten der Formel

HX-CHR³-COOH:

Zu 7 mmmol eines Milchsäurederivates HX-CHR³-COOH in 15 ml trockenem Dimethylsulfoxid werden 1,57 g (14 mmol) Kalium-tert.-butylat zugegeben und 1 Stunde bei Raumtemperatur gerührt. Nach Zugabe von 1,61 g (7 mmol) 2-Methylsulfonyl-4-methyl-5,6-dihydrofuran[2,3-d]pyrimidin wird das Reaktionsgemisch 48 Stunden bei Raumtemperatur gerührt und dann auf 300 ml Wasser, dem 2,5 ml Phosphorsäure zugesetzt sind, gegeben. Man extrahiert mit Essigsäureethylester, trocknet über Natriumsulfat und entfernt das Lösungsmittel im Vakuum. Das Rohrprodukt kann bei Bedarf durch Chromatographie an Silica-Gel gereinigt werden. Handelt es sich um einen Feststoff, so kann er auch durch Umkristallisieren aus einem geeigneten Lösungsmittel weiter gereinigt werden.

Soll ein Milchsäurederivat HX-CHR³-COR¹ umgesetzt werden, bei dem R¹ kein acides Proton trägt, so verwendet man nur ein Aquivalent (7 mmol) Kalium-tert.-butylat.

Gemäß dieser allgemeinen Vorschrift wurden die in nachstehender Tabelle aufgeführten Verbindungen hergestellt.

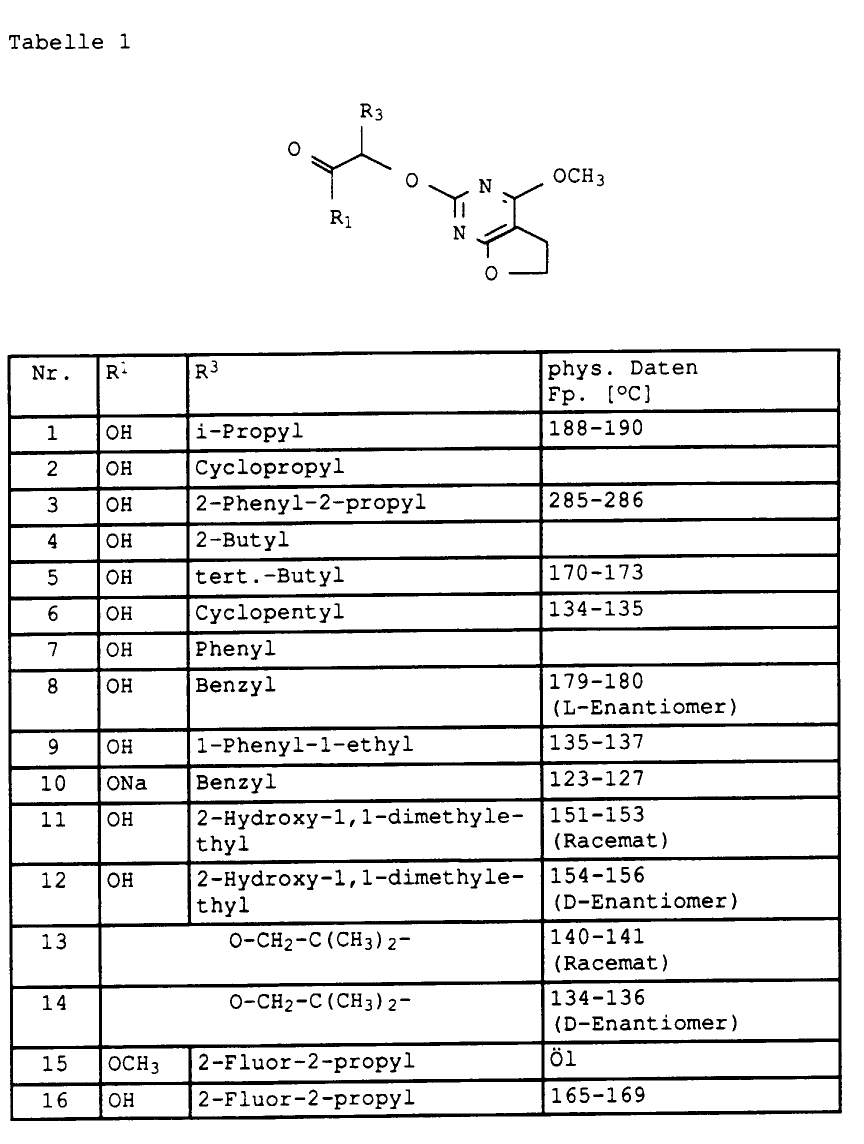

### Anwendungsbeispiele

Die herbizide Wirkung der Verbindungen I ließ sich durch Gewächshausversuche zeigen:

Als Kulturgefäße dienten Plastikblumentöpfe mit lehmigem Sand mit etwa 3 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 und 0,5 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Abkürzung | Deutscher Name |
|---|---|---|
| Glycine max | GLXMA | Sojabohne |
| Avena fatua | AVEFA | Flughafer |
| Amaranthus retroflexus | AMARE | Zurückgekrümmter Fuchsschwanz |
| Stellaria media | STEME | Vogelmiere |
| Sinapis alba | SINAL | Weißer Senf |
| Triticum aestivum | TRZAS | Sommerweizen |

Mit 0,5 bzw. 0,25 kg/ha aktive Substanz erzielt man sehr gute herbizide Wirkung, z.B. mit den Beispielen Nr. 5 und 6. Dabei zeigt die Verbindung 5 eine gute Selektivität in der Beispielkultur Weizen, während Verbindung 6 ausgesprochen selektiv in Soja wirkt.

## Patentansprüche

1. Glykolaldehyd- und Milchsäurederivate sowie deren Schwefelanaloge der Formel I 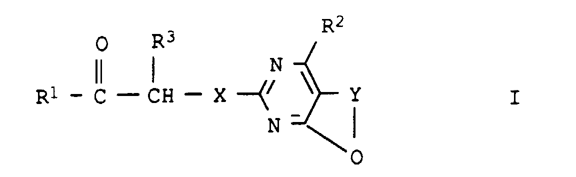 in der die Substituenten folgende Bedeutung haben:
R¹Wasserstoff;
eine Succinylimidoxygruppe;
ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
ein Rest 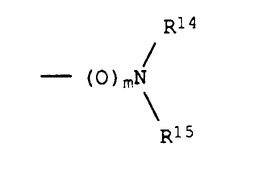 in dem m für 0 oder 1 steht und R¹⁴ und R¹⁵, die gleich oder unterschiedlich sind, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino, cyclo-C₁-C₆-Alkyl, optionell substituiertes Phenyl;
optionell substituiertes cyclo-C₃-C₆-Alkyl;
optionell substituiertes Phenyl;
oder R¹⁴ und R¹⁵ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewahlt aus der Gruppe Sauerstoff, Schwefel oder Stickstoff;
R¹ ferner eine Gruppe 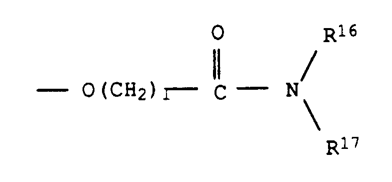 in der R¹⁶ und R¹⁷, die gleich oder unterschiedlich sind, für Wasserstoff, C₁-C₆-Alkyl, optionell substituiertes Phenyl, C₃-C₆-Alkenyl oder C₃-C₆-Alkinyl stehen und l die Werte 1, 2, 3 oder 4 annimmt;
R¹ ferner eine Gruppe 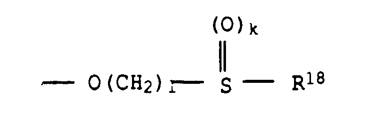 in der R¹⁸ für C₁-C₆-Alkyl, optionell substituiertes Phenyl, C₁-C₆-Halogenalkyl, C₃-C₆-Alkenyl oder C₃-C₆-Alkinyl steht, l die Werte 1, 2, 3 oder 4 und k die Werte 0, 1 oder 2 annehmen;
R¹ ferner einen Rest OR⁵, worin R⁵ bedeutet:
a) Wasserstoff, ein Alkalimetallkation, das Äquivalent eines Erdalkalimetallkations, das Ammoniumkation oder ein organisches Ammoniumion;
b) eine C₃-C₁₂-Cycloalkylgruppe, welche ein bis drei C₁-C₄-Alkylreste tragen kann;
c) eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₃-C₁₂-Cycloalkyl, C₁-C₈-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkythio;
d) eine C₁-C₁₀-Alkylgruppe, welche ein bis fünf Halogenatome tragen kann und einen der folgenden Reste trägt: ein 5-gliedriger Heteroaromat enthaltend ein bis drei Stickstoffatome, oder ein 5-gliedriger Heteroaromat enthaltend ein Stickstoffatom und ein Sauerstoff- oder Schwefelatom, welche ein bis vier Halogenatome und/oder ein bis zwei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
e) eine C₂-C₆-Alkylgruppe, welche in der 2-Position einen der folgenden Reste trägt: C₁-C₆-Alkoxyimino, C₃-C₆-Alkenyloxyimino, C₃-C₆-Halogenalkenyloxyimino oder Benzyloxyimino;
f) eine C₃-C₆-Alkenyl- oder eine C₃-C₆-Alkinylgruppe, wobei diese Gruppen ihrerseits ein bis fünf Halogenatome tragen können;
g) ein Phenylrest, welcher ein bis fünf Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
h) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
i) eine Gruppe -N=CR⁶R⁷, worin R⁶ und R⁷ bedeuten: C₁-C₂₀-Alkyl, welches seinerseits einen Phenylrest, eine C₁-C₄-Alkoxy- und/oder eine C₁-C₄-Alkylthiogruppe tragen kann; Phenyl oder R⁶, R⁷ gemeinsam eine C₃-C₁₂-Alkylenkette, welche ein bis drei C₁-C₃-Alkylgruppen tragen kann;
R¹ ferner ein Rest 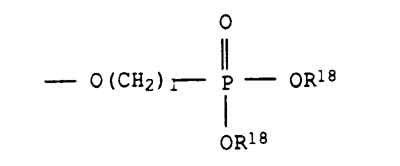 in dem R¹⁸ und l die oben genannte Bedeutung haben,
R¹ ferner ein Rest 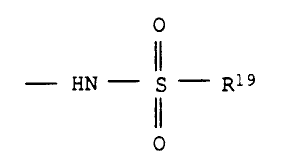 in dem R¹⁹ für die Reste C₁-C₆-Alkyl oder Phenyl steht, die ihrerseits ein bis vier der folgenden Substituenten tragen können:
Halogen, Nitro, Cyano, C₁-C₆-Alkyl;
R² Halogen, C₁-C₄-Alkyl, C₁-C₄-Halogenalkyl, C₁-C₄-Alkoxy, C₁-C₄-Halogenalkoxy oder C₁-C₄-Alkylthio;
R³ Wasserstoff;
eine C₁-C₈-Alkyl-, C₂-C₈-Alkenyl-, C₂-C₈-Alkinyl-, Phenyl-, C₃-C₈-Cycloalkenyl- oder C₃-C₈-Cycloalkylgruppe, die jeweils ein bis fünf Halogenatome und unabhängig voneinander ein bis drei der folgenden Substituenten tragen können:
i) Hydroxy, C₁-C₄-Halogenalkyl, C₁-C₄-Alkoxy, C₁-C₄-Alkylthio, Cyano, Nitro, C₁-C₄-Alkoxycarbonyl, C₁-C₄-Alkylcarbonyl, C₁-C₄-Alkyl, Phenylcarbonyl, C₃-C₁₂-Cycloalkyl, C₃-C₁₂-Cycloalkenyl;
ii) einen 5-gliedrigen Heterocyclus, enthaltend keine, eine oder zwei Doppelbindungen, sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkoxy, C₁-C₄-Alkylthio, C₁-C₄-Halogenalkyl oder Phenyl, das seinerseits ein bis drei Halogenatome und/oder ein bis drei Methylgruppen tragen kann;
iii)einen Thienylrest, der ein bis drei Halogenatome und/oder einen bis drei der folgenden Reste tragen kann: C₁-C₄-Alkyl, C₁-C₂-Halogenalkyl oder Nitro;
iv) einen Pyridylrest, der ein bis drei Halogenatome und/oder einen bis drei der folgenden Reste tragen kann: C₁-C₄-Alkyl, C₁-C₂-Halogenalkyl oder Nitro;
v) einen Naphthyl-, Chinolin-, Benzoxazolyl-, Benzthiazolyl-, Benzthienyl-, Indazolyl- oder Benztriazolylrest, welcher jeweils ein bis drei Halogenatome und/oder einen bis drei der folgenden Reste tragen kann: C₁-C₄-Alkyl oder C₁-C₂-Halogenalkyl;
vi) einen Phenylrest, der seinerseits ein bis fünf Halogenatome und/oder ein bis drei der folgenden Reste tragen kann: C₁-C₄-Alkyl, C₁-C₄-Alkoxy, C₁-C₄-Halogenalkyl, C₁-C₄-Halogenalkoxy, Cyano, Nitro, C₁-C₄-Dialkylamino, und/oder C₁-C₄-Alkylthio;
vii) einen 5- oder 6-gliedrigen Heterocyclus, enthaltend keine, eine oder zwei Doppelbindungen sowie ein bis zwei Sauerstoff- oder Schwefelatome, der außerdem folgende Reste tragen kann: C₁-C₄-Alkyl, C₁-C₄-Alkoxy, C₁-C₄-Halogenalkyl, C₁-C₄-Halogenalkoxy oder Nitro;
R³ ferner einen 5- oder 6-gliedrigen Heterocyclus, enthaltend keine, eine oder zwei Doppelbindungen, sowie ein bis vier Stickstoffatome oder ein bis zwei Stickstoffatome sowie zusätzlich ein Schwefel- oder Sauerstoffatom, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl, C₁-C₄-Halogenalkoxy, C₁-C₄-Alkoxy oder Phenyl, das seinerseits ein bis drei Halogenatome und/oder ein bis drei Methylgruppen tragen kann;
einen Pyridylrest, der ein bis drei Halogenatome und/oder einen bis drei der folgenden Reste tragen kann: C₁-C₄-Alkyl, C₁-C₂-Halogenalkyl oder Nitro;
einen Naphthyl-, Chinolin-, Benzoxazolyl-, Indazolyl- oder Benztriazolylrest, welcher jeweils ein bis drei Halogenatome und/oder einen bis drei der folgenden Reste tragen kann: C₁-C₄-Alkyl oder C₁-C₂-Halogenalkyl;
einen 5- oder 6-gliedrigen Heterocyclus, enthaltend keine, eine oder zwei Doppelbindungen sowie ein bis zwei Sauerstoff- oder Schwefelatome, der außerdem folgende Reste tragen kann: Halogen, Nitro, C₁-C₄-Alkyl, C₁-C₄-Alkoxy, C₁-C₄-Halogenalkyl, C₁-C₄-Halogenalkoxy;
R³ gemeinsam mit R¹ eine optionell substituierte C₃-C₅-Alkylenkette, in der eine CH₂-Gruppe durch Sauerstoff, Schwefel oder Stickstoff ersetzt sein kann;
X ein Sauerstoffatom, ein Schwefelatom oder eine Einfachbindung;
Y eine C₂-C₄-Alkylen oder C₂-C₄-Alkenylenkette, wobei eine Methylengruppe jeweils durch eine Oxo-Gruppe (=O) substituiert sein kann und/oder die Alkylen- bzw. Alkenylenkette durch C₁-C₄-Alkyl, Phenyl, C₁-C₄-Alkoxy oder C₁-C₄-Alkoxycarobnyl substituiert sein kann;
wobei in den oben genannten Fällen der Ausdruck optionell substituiert jeweils bedeutet, daß die so bezeichneten Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio,
sowie umweltverträgliche Salze der Verbindungen I.

2. Glykolaldehyd- und Milchsäurederivate der Formel I gemäß Anspruch 1, in der R² Methoxy und X Sauerstoff bedeuten und R¹ und R³ die in Anspruch 1 genannte Bedeutung haben.

3. Milchsäurederivate der Formel I gemäß Anspruch 1, in der R¹ eine Gruppe OR⁵ bedeutet und R⁵ für Wasserstoff, C₁-C₁₀-Alkyl, Benzyl, C₃-C₆-Alkenyl oder C₃-C₆-Alkinyl steht, R² Methoxy, R³ Wasserstoff oder C₁-C₈-Alkyl, das wie in Anspruch 1 genannt substituiert sein kann, X Sauerstoff oder Schwefel und Y eine C₂H₄-Kette bedeuten.

4. Milchsäurederivate der Formel I gemäß Anspruch 1, in der R¹ eine Gruppe OR⁵ bedeutet und R⁵ für eine Gruppe -N=CR⁶R⁷ steht, in der R⁶ bzw. R⁷ einen C₁-C₄-Alkylrest, der unsubstituiert oder durch Phenyl, C₁-C₄-Alkoxy und/oder C₁-C₄-Alkylthio substituiert ist, oder einen Phenylrest darstellt oder R⁶ zusammen mit R⁷ eine C₃-C₆-Alkylenkette bildet, die durch C₁-C₃-Alkyl substituiert sein kann, R² Methoxy, R³ Wasserstoff oder C₁-C₈-Alkyl, das wie in Anspruch 1 genannt substituiert sein kann, X Sauerstoff oder Schwefel und Y eine C₂H₄-Kette bedeuten.

5. Herbizides Mittel oder Mittel zur Beeinflussung des Pflanzenwachstums, enthaltend eine Verbindung der Formel I gemäß Anspruch 1 und übliche inerte Zusatzstoffe..

6. Herbizides Mittel oder Mittel zur Beeinflussung des Pflanzenwachstums, enthaltend und übliche inerte Zusatzstoffe und eine Verbindung der Formel I gemäß Anspruch 1, in der R¹ eine Gruppe OR⁵ bedeutet und R⁵ für Wasserstoff, C₁-C₁₀-Alkyl, Benzyl, C₃-C₆-Alkenyl oder C₃-C₆-Alkinyl steht, R² Methoxy, R³ Wasserstoff oder C₁-C₈-Alkyl, das wie in Anspruch 1 genannt substituiert sein kann, X Sauerstoff oder Schwefel und Y eine C₂H₄-Kette bedeuten.

7. Verfahren zur Bekämpfung unerwünschten Pflanzenwuchses, dadurch gekennzeichnet, daß man eine herbizid wirksame Menge einer Verbindung der Formel I gemäß Anspruch 1 auf die Pflanzen oder deren Lebensraum einwirken läßt.

8. Verfahren zur Regulierung des Pflanzenwachstums, dadurch gekennzeichnet, daß man eine bioregulatorisch wirksame Menge einer Verbindung der Formel I gemäß Anspruch 1 auf die Pflanzen oder deren Lebensraum einwirken läßt.

9. Verfahren zur Herstellung der Glykolaldehyd- und Milchsäurederivate der Formel I gemäß Anspruch 1, dadurch gekennzeichnet, daß man Glykolaldehyd- bzw. Milchsäurederivate der Formel II 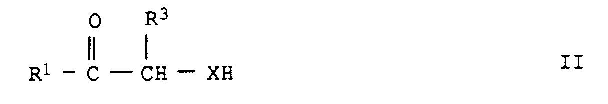 mit Sulfonen der Formel III 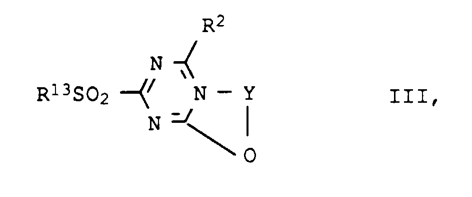 wobei die Reste R¹ bis R³, X und Y die in Anspruch 1 genannte Bedeutung haben und R¹³ SO₂ eine übliche nukleofuge Abgangsgruppe darstellt, in Gegenwart einer Base umsetzt.

## Claims

1. A glycol aldehyde or lactic acid derivative or a sulfur analog thereof of the formula I 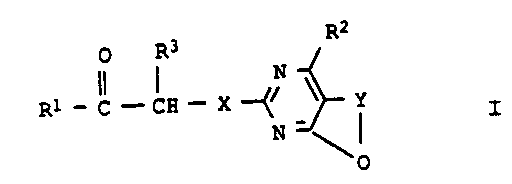 where
R¹ is hydrogen;
succinylimidoxy;
a 5-membered heteroaromatic structure which is bonded via a nitrogen atom, contains two or three nitrogen atoms and may carry one or two halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio; a radical 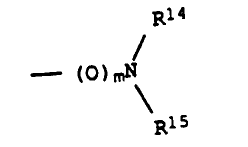 in which m is 0 or 1 and R¹⁴ and R¹⁵ are identical or different and are each
hydrogen;
C₁-C₆-alkyl, C₃-C₆-alkenyl or C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxyarbonyyl C₃-C₆-alkenyloxycarbonyl, C₃-C₆-alkynyloxycarbonyl, bis-C₁-C₆-dialkylamino, cyclo-C₁-C₆-alkyl or unsubstituted or substituted phenyl; unsubstituted or substituted cyclo-C₃-C₆-alkyl;
or unsubstituted or substituted phenyl;
or R¹⁴ together with R¹⁵ form an unsubstituted or substituted, cyclized C₄-C₇-alkylene chain or together form an unsubstituted or substituted, cyclized C₃-C₆-alkylene chain having a hetero atom selected from the group consisting of oxygen, sulfur and nitrogen; R¹ is furthermore a group 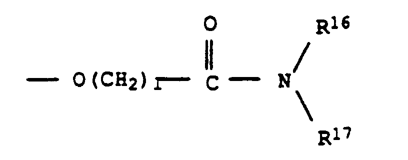 where R¹⁶ and R¹⁷ are identical or different and are each hydrogen, C₁-C₆-alkyl, unsubstituted or substituted phenyl, C₃-C₆-alkenyl or C₃-C₆-alkynyl and 1 is 1, 2, 3 or 4;
R¹ is furthermore a group 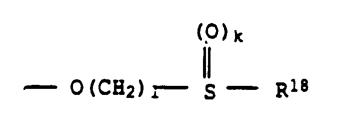 where R¹⁸ is C₁-C₆-alkyl, unsubstituted or substituted phenyl, C₁-C₆-haloalkyl, C₃-C₆-alkenyl or C₃-C₆-alkynyl, 1 is 1, 2, 3 or 4 and k is 0, 1 or 2;
R¹ is furthermore OR⁵, where R⁵ is
(a) hydrogen, an alkali metal cation, one equivalent of an alkaline earth metal cation, the ammonium cation or an organic ammonium ion;
(b) C₃-C₁₂-cycloalkyl which may carry from one to three C₁-C₄-alkyl radicals;
(c) C₁-C₁₀-alkyl which may carry from one to five halogen atoms and/or one of the following radicals: C₁-C₄-alkoxy, C₁-C₄-alkylthio, cyano, C₁-C₈-alkylcarbonyl, C₃-C₁₂-cycloalkyl, C₁-C₈-alkoxycarbonyl, phenyl, phenoxy or phenylcarbonyl, where the aromatic radicals in turn may each carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
(d) C₁-C₁₀-alkyl which may carry from one to five halogen atoms and carries one of the following radicals: a 5-membered heteroaromatic structure containing from one to three nitrogen atoms or a 5-membered heteroaromatic structure containing one nitrogen atom and one oxygen or sulfur atom, which may carry from one to four halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
(e) C₂-C₆-alkyl which carries one of the following radicals in the 2-position: C₁-C₆-alkoximino, C₃-C₆-alkenyloximino, C₃-C₆-haloalkenyloximino or benzyloximino;
(f) C₃-C₆-alkenyl or C₃-C₆-alkynyl, where these groups in turn may carry from one to five halogen atoms;
(g) phenyl which may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
(h) a 5-membered heteroaromatic structure which is bonded via a nitrogen atom, contains from one to three nitrogen atoms and may carry one or two halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
(i) a group -N=CR⁶R⁷, where R⁶ and R⁷ are each
C₁-C₂₀-alkyl which in turn may carry phenyl, C₁-C₄-alkoxy and/or C₁-C₄-alkylthio; or phenyl; or R⁶ and R7 together form a C₃-C₁₂-alkylene chain which may carry from one to three C₁-C₃-alkyl groups;
R¹ is furthermore a radical 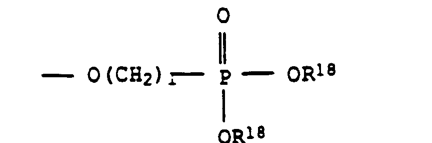 where R¹⁸ and l have the abovementioned meanings,
R¹ is furthermore a radical 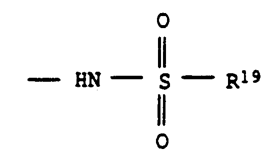 where R¹⁹ is C₁-C₆-alkyl or phenyl which in turn may carry from one to four of the following substituents: halogen, nitro, cyano, C₁-C₆-alkyl;
R² is halogen, C₁-C₄-alkyl, C₁-C₄-haloalkyl, C₁-C₄-alkoxy, C₁-C₄-haloalkoxy or C₁-C₄-alkylthio;
R³ is hydrogen;
C₁-C₈-alkyl, C₂-C₈-alkenyl, C₂-C₈-alkynyl, phenyl, C₃-C₈-cycloalkenyl or C₃-C₈-cycloalkyl, each of which may carry from one to five halogen atoms and, independently of one another, from one to three of the following substituents:
(i) hydroxyl, C₁-C₄-haloalkyl, C₁-C₄-alkoxy, C₁-C₄-alkylthio, cyano, nitro, C₁-C₄-alkoxycarbonyl, C₁-C₄-alkylcarbonyl, C₁-C₄-alkyl, phenylcarbonyl, C₃-C₁₂-cycloalkyl, C₃-C₁₂-cycloalkenyl;
(ii) a 5-membered heterocyclic structure containing no double bonds or one or two double bonds and from one to four nitrogen atoms or one or two nitrogen atoms and additionally one sulfur or oxygen atom, which may carry from one to three halogen atoms and/or from one to three of the following radicals: nitro, cyano, C₁-C₄-alkyl, C₁-C₄-alkoxy, C₁-C₄-alkylthio, C₁-C₄-haloalkyl or phenyl, which in turn may carry from one to three halogen atoms and/or from one to three methyl groups;
(iii) thienyl which may carry from one to three halogen atoms and/or from one to three of the following radicals: C₁-C₄-alkyl, C₁- or C₂-haloalkyl or nitro;
(iv) pyridyl which may carry from one to three halogen atoms and/or from one to three of the following radicals: C₁-C₄-alkyl, C₁- or C₂-haloalkyl or nitro;
(v) naphthyl, quinolyl, benzoxazolyl, benzothiazolyl, benzothienyl, indazolyl or benzotriazolyl, each of which may carry from one to three halogen atoms and/or from one to three of the following radicals: C₁-C₄-alkyl or C₁- or C₂-haloalkyl;
(vi) phenyl which in turn may carry from one to five halogen atoms and/or from one to three of the following radicals: C₁-C₄-alkyl, C₁-C₄-alkoxy, C₁-C₄-haloalkyl, C₁-C₄-haloalkoxy, cyano, nitro, C₁-C₄-dialkylamino and/or C₁-C₄-alkylthio;
(vii) a 5-membered or 6-membered heterocyclic structure containing no double bonds or one or two double bonds and one or two oxygen or sulfur atoms, which may furthermore carry the following radicals: C₁-C₄-alkyl, C₁-C₄-alkoxy, C₁-C₄-haloalkyl, C₁-C₄-haloalkoxy or nitro;
R³ may furthermore be a 5-membered or 6-membered heterocyclic structure containing no double bonds or one or two double bonds and from one to four nitrogen atoms or one or two nitrogen atoms and additionally one sulfur or oxygen atom, which may carry from one to three halogen atoms and/or from one to three of the following radicals: nitro, cyano, C₁-C₄-alkyl, C₁-C₄-alkylthio, C₁-C₄-haloalkyl, C₁-C₄-haloalkoxy, C₁-C₄-alkoxy or phenyl, which in turn may carry from one to three halogen atoms and/or from one to three methyl groups;
pyridyl which may carry from one to three halogen atoms and/or from one to three of the following radicals: C₁-C₄-alkyl, C₁- or C₂-haloalkyl or nitro;
naphthyl, quinolyl, benzoxazolyl, indazolyl or benzotriazolyl, each of which may carry from one to three halogen atoms and/or from one to three of the following radicals: C₁-C₄-alkyl or C₁- or C₂-haloalkyl;
a 5-membered or 6-membered heterocyclic structure containing no double bonds or one or two double bonds and one or two oxygen or sulfur atoms, which may furthermore carry the following radicals: halogen, nitro, C₁-C₄-alkyl, C₁-C₄-alkoxy, C₁-C₄-haloalkyl or C₁-C₄-haloalkoxy;
R³ together with R¹ is an unsubstituted or substituted C₃-C₅-alkylene chain where CH₂ may be replaced by oxygen, sulfur or nitrogen;
X is oxygen, sulfur or a single bond and
Y is a C₂-C₄-alkylene or C₂-C₄-alkenylene chain where in each case a methylene group may be substituted by an oxo group (=O) and/or where the alkylene or alkenylene chain may be substituted by C₁-C₄-alkyl, phenyl, C₁-C₄-alkoxy or C₁-C₄-alkoxycarbonyl;
in the abovementioned cases the expression unsubstituted or substituted meaning in each case that the groups so referred to may carry one or more of the following substituents: halogen, nitro, cyano, C₁-C₆-alkyl, C₁-C₆-haloalkyl, C₁-C₆-alkoxy and C₁-C₆-alkylthio,
and environmentally compatible salts of the compound I.

2. A glycol aldehyde or lactic acid derivative of the formula I as claimed in claim 1, wherein R² is methoxy, X is oxygen and R¹ and R³ have the meanings stated in claim 1.

3. A lactic acid derivative of the formula I as claimed in claim 1, wherein R¹ is OR⁵, R⁵ is hydrogen, C₁-C₁₀-alkyl, benzyl, C₃-C₆-alkenyl or C₃-C₆-alkynyl, R² is methoxy, R³ is hydrogen or C₁-C₈-alkyl which may be substituted as stated in claim 1, X is oxygen or sulfur and Y is a C₂H₄ chain.

4. A lactic acid derivative of the formula I as claimed in claim 1, wherein R¹ is OR⁵, R⁵ is a group -N=CR⁶R⁷, where R⁶ and R⁷ are each C₁-C₄-alkyl which is unsubstituted or substituted by phenyl, C₁-C₄-alkoxy and/or C₁-C₄-alkylthio, or are each phenyl, or R⁶ together with R⁷ forms a C₃-C₆-alkylene chain which may be substituted by C₁-C₃-alkyl, R² is methoxy, R³ is hydrogen or C₁-C₈-alkyl which may be substituted as stated in claim 1, X is oxygen or sulfur and Y is a C₂H₄ chain.

5. A herbicide or plant growth regulator containing a compound of the formula I as claimed in claim 1 and conventional inert additives.

6. A herbicide or plant growth regulator containing conventional inert additives and a compound of the formula I as claimed in claim 1 where R¹ is OR⁵, R⁵ is hydrogen, C₁-C₁₀-alkyl, benzyl, C₃-C₆-alkenyl or C₃-C₆-alkynyl, R² is methoxy, R³ is hydrogen or C₁-C₈-alkyl which may be substituted as stated in claim 1, X is oxygen or sulfur and Y is a C₂H₄ chain.

7. A method for controlling undesirable plant growth, wherein a herbicidal amount of a compound of the formula I as claimed in claim 1 is allowed to act on the plants or on their habitat.

8. A method for regulating plant growth, wherein a bioregulatory amount of a compound of the formula I as claimed in claim 1 is allowed to act on the plants or on their habitat.

9. A process for the preparation of a glycol aldehyde or lactic acid derivative of the formula I as claimed in claim 1, wherein a glycol aldehyde or lactic acid derivative of the formula II 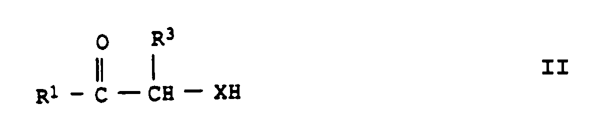 is reacted with a sulfone of the formula III 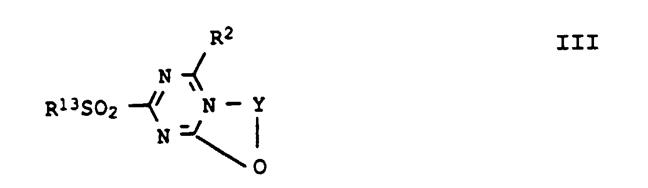 where R¹ to R³, X and Y have the meanings stated in claim 1 and R¹³SO₂ is a conventional nucleofugic leaving group, in the presence of a base.

## Revendications

1. Dérivés de l'aldéhyde glycolique et de l'acide lactique, et leurs analogues sulfurés, répondant à la formule I 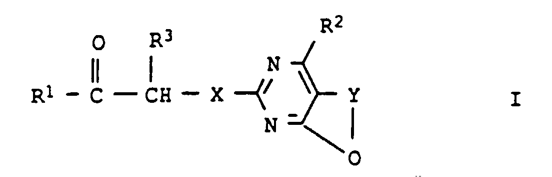 dans laquelle les symboles ont les significations suivantes :
R¹ représente l'hydrogène ;
un groupe succinylimidoxy ;
un noyau hétéroaromatique à cinq chaînons relié par un atome d'azote, contenant deux à trois atomes d'azote et qui peut porter un à deux atomes d'halogènes et/ou un à deux des groupes suivants : alkyle en C1-C4, halogénoalkyle en C1-C4, alcoxy en C1-C4, halogénoalcoxy en C1-C4 et/ou alkylthio en C1-C4 ;
un groupe 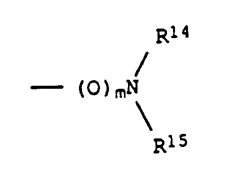 dans lequel m est égal à 0 ou 1 et R¹⁴ et R¹⁵, qui peuvent avoir des significations identiques ou différentes, représentent :
l'hydrogène ;
un groupe alkyle en C1-C6, alcényle en C3-C6, alcynyle en C3-C6, ces groupes pouvant chacun porter un à cinq atomes d'halogènes et/ou un à deux des substituants suivants :
alcoxy en C1-C6, alcényloxy en C3-C6, alcynyloxy en C3-C6, alkylthio en C1-C6, alcénylthio en C3-C6, alcynylthio en C3-C6, halogénoalcoxy en C1-C6, cyano, (alkyle en C1-C6)-carbonyle, (alcényle en C3-C6)carbonyle, (alcynyle en C3-C6)carbonyle, (alcoxy en Cl-C6)carbonyle, (alcényloxy en C3-C6)carbonyle, (alcynyloxy en C3-C6)carbonyle, bis-(dialkyle en C1-C6)amino, cycloalkyle en C1-C6, phényle éventuellement substitué ;
un groupe cycloalkyle en C1-C6 éventuellement substitué ;
un groupe phényle éventuellement substitué ;
ou bien R¹⁴ et R¹⁵ représentent, ensemble, une chaîne alkylène en C4-C7 cyclisée et éventuellement substituée, ou bien une chaîne alkylène en C3-C6 cyclisée et éventuellement substituée, contenant un hétéroatome choisi parmi l'oxygène, le soufre ou l'azote ;
R¹ peut en outre représenter un groupe 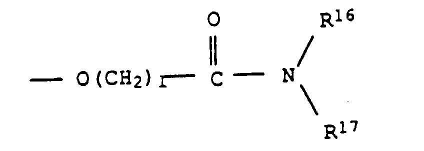 dans lequel R¹⁶ et R¹⁷, qui peuvent avoir des significations identiques ou différentes, représentent chacun l'hydrogène, un groupe alkyle en C1-C6, un groupe phényle éventuellement substitué, un groupe alcényle en C3-C6 ou alcynyle en C3-C6 et 1 est égal à 1, 2, 3 ou 4 ;
R¹ peut en outre représenter un groupe 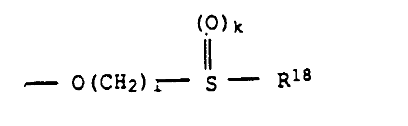 dans lequel R¹⁸ représente un groupe alkyle en C1-C6, un groupe phényle éventuellement substitué, un groupe halogénoalkyle en C1-C6, alcényle en C3-C6 ou alcynyle en C3-C6, et 1 est égal à 1, 2, 3 ou 4 et k à 0, 1 ou 2 ;
R¹ peut en outre représenter un groupe OR⁵ dans lequel R⁵ représente
a) l'hydrogène, un cation de métal alcalin, l'équivalent d'un cation de métal alcalino-terreux, le cation ammonium ou un ion ammonium organique ;
b) un groupe cycloalkyle en C3-C12 qui peut porter un à trois groupes alkyle en C1-C4 ;
c) un groupe alkyle en C1-C10 qui peut porter un à cinq atomes d'halogènes et/ou un des substituants suivants : alcoxy en C1-C4, alkylthio en C1-C4, cyano, (alkyle en C1-C8)carbonyle, cycloalkyle en C3-C12, (alcoxy en C1-C8)carbonyle, phényle, phénoxy ou phénylcarbonyle, les radicaux aromatiques pouvant eux-mêmes porter chacun un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
d) un groupe alkyle en C1-C10 qui peut porter un à cinq atomes d'halogènes et un des substituants suivants : un cycle hétéroaromatique à cinq chaînons contenant un à trois atomes d'azote, ou un cycle hétéroaromatique à cinq chaînons contenant un atome d'azote et un atome d'oxygène ou de soufre, qui peuvent porter un à quatre atomes d'halogènes et/ou un à deux des substituants suivants : alkyle en C1-C4, halogénoalkyle en C1-C4, alcoxy en C1-C4, halogénoalcoxy en C1-C4 et/ou alkylthio en C1-C4 ;
e) un groupe alkyle en C2-C6 qui porte en position 2 l'un des substituants suivants : alcoxyimino en C1-C6, alcényloxyimino en C3-C6, halogénoalcényloxyimino en C3-C6 ou benzyloxyimino ;
f) un groupe alcényle en C3-C6 ou alcynyle en C3-C6, ces groupes pouvant lui-mêmes porter un à cinq atomes d'halogènes ;
g) un groupe phényle qui peut porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
h) un cycle hétéroaromatique à cinq chaînons relié par l'atome d'azote, contenant un à trois atomes d'azote et qui peut porter un à cinq atomes d'halogènes et/ou un à deux des substituants suivants : alkyle en C1-C4, halogénoalkyle en C1-C4, alcoxy en C1-C4, halogénoalcoxy en C1-C4 et/ou alkylthio en C1-C4 ;
i) un groupe -N=CR⁶R⁷ dans lequel R⁶ et R⁷ représentent :
des groupes alkyle en C1-C20 qui peuvent porter eux-mêmes un groupe phényle, un groupe alcoxy en C1-C4 et/ou un groupe alkylthio en C1-C4 ; des groupes phényle, ou bien R⁶ et R⁷ forment ensemble une chaîne alkylène en C3-C12 qui peut porter un à trois groupes alkyle en C1-C3 ;
R¹ peut en outre représenter un groupe 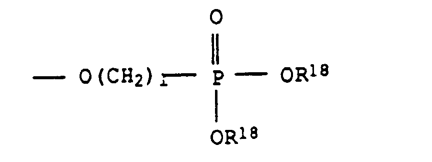 dans lequel R¹⁸ et 1 ont les significations indiquées ci-dessus,
R¹ peut en outre représenter un groupe 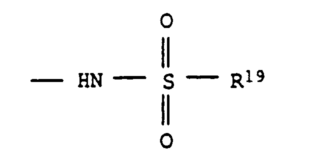
dans lequel R¹⁹ représente un groupe alkyle en C1-C6 ou phényle, qui peuvent eux-mêmes porter un à quatre des substituants suivants :
des halogènes, des groupes nitro, cyano, alkyle en C1-C6 ;
R² représente un halogène, un groupe alkyle en C1-C4, halogénoalkyle en C1-C4, alcoxy en C1-C4, halogénoalcoxy en C1-C4 ou alkylthio en C1-C4 ;
R³ représente l'hydrogène ;
un groupe alkyle en C1-C8, alcényle en C2-C8, alcynyle en C2-C8, phényle, cycloalcényle en C3-C8 ou cycloalkyle en C3-C8, qui peuvent porter chacun un à cinq atomes d'halogènes et, indépendamment les uns des autres, un à trois des substituants suivants :
i) hydroxy, halogénoalkyle en C1-C4, alcoxy en C1-C4, alkylthio en C1-C4, cyano, nitro, (alcoxy en C1-C4)carbonyle, (alkyle en C1-C4)carbonyle, alkyle en C1-C4, phénylcarbonyle, cycloalkyle en C3-C12, cycloalcényle en C3-C12 ;
ii) un hétérocycle à cinq chaînons sans ou contenant une ou deux doubles liaisons et un à quatre atomes d'azote ou un à deux atomes d'azote et en outre un atome de soufre ou d'oxygène, et qui peut porter un à trois atomes d'halogènes et/ou un à trois des substituants suivants : nitro, cyano, alkyle en C1-C4, alcoxy en C1-C4, alkylthio en C1-C4, halogénoalkyle en C1-C4 ou phényle, lequel peut porter un à trois atomes d'halogènes et/ou un à trois groupes méthyle ;
iii) un groupe thiényle qui peut porter un à trois atomes d'halogènes et/ou un à trois des substituants suivants : alkyle en C1-C4, halogénoalkyle en C1-C2 ou nitro ;
iv) un groupe pyridyle qui peut porter un à trois atomes d'halogènes et/ou un à trois des substituants suivants : alkyle en C1-C4, halogénoalkyle en C1-C2 ou nitro ;
v) un groupe naphtyle, quinoléinyle, benzoxazolyle, benzothiozolyle, benzothiényle, indazolyle ou benzotriazolyle, qui peuvent chacun porter un à trois atomes d'halogènes et/ou un à trois des substituants suivants : alkyle en C1-C4 ou halogénoalkyle en C1-C2;
vi) un groupe phényle qui peut lui-même porter un à cinq atomes d'halogènes et/ou un à trois des substituants suivants : alkyle en C1-C4, alcoxy en C1-C4, halogénoalkyle en C1-C4, halogénoalcoxy en C1-C4, cyano, nitro, di-(alkyle en C1-C4)amino et/ou alkylthio en C1-C4;
vii) un hétérocycle à cinq ou six chaînons sans ou avec une ou deux doubles liaisons et un à deux atomes d'oxygène ou de soufre, qui peut en outre porter les substituants suivants : alkyle en C1-C4, alcoxy en C1-C4, halogénoalkyle en C1-C4, halogénoalcoxy en C1-C4 ou nitro ;
R³ peut en outre représenter un hétérocycle à cinq ou six chaînons sans ou avec une ou deux doubles liaisons et un à quatre atomes d'azote ou un à deux atomes d'azote et en outre un atome de soufre ou d'oxygène, qui peut porter un à trois atomes d'halogènes et/ou un à trois des substituants suivants : nitro, cyano, alkyle en C1-C4, alkylthio en C1-C4, halogénoalkyle en C1-C4, halogénoalcoxy en C1-C4, alcoxy en C1-C4 ou phényle pouvant porter lui-même un à trois atomes d'halogènes et/ou un à trois groupes méthyle ;
un groupe pyridyle qui peut porter un à trois atomes d'halogènes et/ou un à trois des substituants suivants : alkyle en C1-C4, halogénoalkyle en C1-C2 ou nitro ;
un groupe naphtyle, quinoléinyle, benzoxazolyle, indazolyle ou benzotriazolyle qui peuvent chacun porter un à trois atomes d'halogènes et/ou un à trois des substituants suivants : alkyle en C1-C4 ou halogénoalkyle en C1-C2 ;
un hétérocycle à cinq à six chaînons sans ou avec une ou deux doubles liaisons et qui contient un à deux atomes d'oxygène ou de soufre et peut en outre porter les substituants suivants : halogéno, nitro, alkyle en C1-C4, alcoxy en C1-C4, halogénoalkyle en C1-C4, halogénoalcoxy en C1-C4 ;
R³ peut également former ensemble avec R¹ une chaîne alkylène en C3-C5 éventuellement substituée et dans laquelle un groupe CH₂ peut être remplacé par l'oxygène, le soufre ou l'azote ;
X représente un atome d'oxygène, un atome de soufre ou une liaison simple ;
Y représente une chaîne alkylène en C2-C4 ou alcénylène en C2-C4, dans laquelle un groupe méthyle peut être remplacé par un groupe oxo (=0) et/ou qui peut être substituée par des groupes alkyle en C1-C4, phényle, alcoxy en C1-C4 ou (alcoxy en Cl -C4)carbonyle ;
étant précisé que l'expression "éventuellement substitué", telle qu'utilisée ci-dessus, indique dans chaque cas que le groupe ainsi qualifié peut porter un ou plusieurs des substituants suivants : halogéno, nitro, cyano, alkyle en C1-C6, halogénoalkyle en C1-C6, alcoxy en C1-C6, alkylthio en C1-C6,
et les sels non polluants des composés I.

2. Dérivés de l'aldéhyde glycolique et de l'acide lactique répondant à la formule I selon la revendication 1, dans laquelle R² représente un groupe méthoxy et X l'oxygène, et R¹ et R³ ont les significations indiquées dans la revendication 1.

3. Dérivés de l'acide lactique de formule selon la revendication 1, dans laquelle R¹ représente un groupe OR⁵ et R⁵ représente l'hydrogène, un groupe alkyle en C1-C10, benzyle, alcényle en C3-C6 ou alcynyle en C3-C6, R² représente un groupe méthoxy, R³ l'hydrogène ou un groupe alkyle en C1-C8 qui peut être substitué comme indiqué dans la revendication 1, X représente l'oxygène ou le soufre et Y une chaîne C₂H₄.

4. Dérivés de l'acide lactique de formule I selon la revendication 1, dans laquelle R¹ représente un groupe OR⁵ et R⁵ représente un groupe -N-CR⁶R⁷ dans lequel R⁶ et R⁷ représentent chacun un groupe alkyle en C1-C4 non substitué ou substitué par des groupes phényle, alcoxy en C1-C4 et/ou alkylthio en C1-C4, ou bien un groupe phényle, ou bien R⁶ forme avec R⁷ une chaîne alkylène en C3-C6 qui peut être substituée par un groupe alkyle en C1-C3, R² représente un groupe méthoxy, R³ l'hydrogène ou un groupe alkyle en C1-C8 qui peut être substitué comme indiqué dans la revendication 1, X représente l'oxygène ou le soufre et Y une chaîne C₂H₄.

5. Produit herbicide ou produit pour agir sur la croissance des végétaux, contenant un composé de formule I selon la revendication 1, et des additifs inertes usuels.

6. Produit herbicide ou produit pour agir sur la croissance des végétaux, contenant des additifs inertes usuels et un composé de formule I selon la revendication 1, dans laquelle R¹ représente un groupe OR⁵ et R⁵ l'hydrogène, un groupe alkyle en C1-C10, benzyle, alcényle en C3-C6 ou alcynyle en C3-C6, R² représente un groupe méthoxy, R³ l'hydrogène ou un groupe alkyle en C1-C8 qui peut être substitué comme indiqué dans la revendication 1, X représente l'oxygène ou le soufre et Y une chaîne C₂H₄.

7. Procédé pour combattre les croissances de végétaux indésirables, caractérisé par le fait que l'on fait réagir une quantité herbicide efficace d'un composé de formule I selon la revendication 1 sur les végétaux ou leur habitat.

8. Procédé pour la régulation de la croissance des végétaux, caractérisé par le fait que l'on fait agir une quantité biorégulatrice efficace d'un composé de formule I selon la revendication 1 sur les végétaux ou leur habitat.

9. Procédé de préparation des dérivés de l'aldéhyde glycolique et de l'acide lactique de formule I selon la revendication 1, caractérisé par le fait que l'on fait réagir des dérivés de l'aldéhyde glycolique ou de l'acide lactique répondant à la formule II 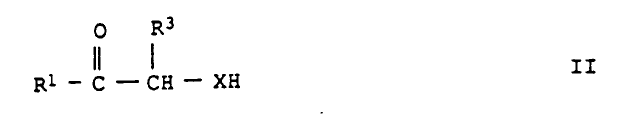 avec des sulfones de formule III 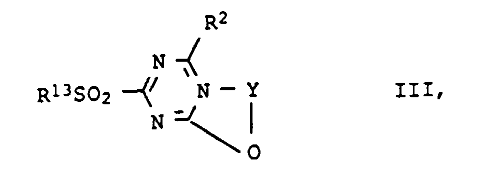 dans lesquelles R¹ à R³, X et Y ont les significations indiquées dans la revendication 1 et R¹³SO₂ représente un groupe éliminable nucléofuge usuel, en présence d'une base.
